(19) Europäisches Patentamt
European Patent Office
Office européen des brevets

(11) EP 0 518 445 B1

(12) EUROPEAN PATENT SPECIFICATION

(45) Date of publication and mention of the grant of the patent: **17.04.1996 Bulletin 1996/16**

(51) Int. Cl.$^6$: **A23L 3/3571**, A23C 19/032, C12N 11/02, A23C 19/06

(21) Application number: **92201722.3**

(22) Date of filing: **11.06.1992**

(54) **Longterm antimicrobial activity obtained by sustained release of hydrogen peroxide**

Langhaltende antimikrobielle Wirkung durch verzögerte Freisetzung von Wasserstoffperoxyd

Activité antimicrobienne prolongée par la libération retardée du péroxyde d'hydrogène

(84) Designated Contracting States:
**AT BE CH DE DK ES FR GB GR IT LI LU MC NL PT SE**

(30) Priority: **11.06.1991 EP 91201442**

(43) Date of publication of application: **16.12.1992 Bulletin 1992/51**

(73) Proprietor: **GIST-BROCADES N.V.**
**NL-2600 MA Delft (NL)**

(72) Inventors:
- **De Jong, Sylvia Josefine**
  **NL-2613 ZC Delft (NL)**
- **De Haan, Ben Rudolf**
  **NL-2272 EN Voorburg (NL)**
- **Tan, Hong Sheng**
  **NL-2665 TG Bleiswijk (NL)**

(74) Representative: **Matulewicz, Emil Rudolf Antonius, Dr. et al**
**Gist-Brocades NV**
**Patents and Trademarks Department**
**Wateringseweg 1**
**P.O. Box 1**
**NL-2600 MA Delft (NL)**

(56) References cited:
**EP-A- 0 397 227**
**EP-A- 0 397 228**
**FR-A- 2 153 443**
**FR-A- 2 237 589**

- **CHEMICAL ABSTRACTS, vol. 113, no. 24, 10 December 1990, Columbus, Ohio, US; abstract no. 214158h & ANALYST, vol.115, no.10, 1990, London GB, pages 1289-1295**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 518 445 B1

## Description

### Technical field

The present invention relates to an improvement in the use of an antimicrobial system. Specifically, the invention relates to the sustained release of hydrogen peroxide. The hydrogen peroxide produced may be used as such or it may be used in combination with suitable reactants to produce substances with antimicrobial activity, specifically hypothiocyanate.

### Background of the invention

Microbial contamination of food and feed can cause severe health problems. Recent examples are the different outbreaks of human listeriosis that have been reported in Canada (Schlech et al. 1983. N.Engl.J.Med. 308 : 203-206), in the United States (Fleming et al. 1985. N.Engl.J.Med. 312 : 404-407 and Linnan et al. 1988. N.Engl.J.Med. 319 : 823-828) and in Switzerland (Food Chem. News. 1987. Dec, 7).

Microbial contamination can also adversely affect products containing proteins or other microbially degradable components.

Different methods to prevent microbial contamination of susceptible products are known such as, chemical methods (addition of compounds such as sulphite, nitrite, benzoic acid, sorbic acid) and the use of bacteriocins. Due to the suspected and proven side-effects of the chemicals used in the chemical methods, the acceptability of such methods is becoming more and more questionable. Furthermore, the applicability of bacteriocins is restricted due to the relatively high specificity of these molecules toward specific microorganisms. This would necessitate the use of mixtures of many different bacteriocins in order to be effective against microorganisms.

The disadvantages of the above-mentioned methods stimulated the search for more acceptable methods. One way to avoid the above problems is the use of naturally occurring antimicrobial systems. Turning the attention to natural mechanisms for preventing microbial growth, an antimicrobial system in milk was identified to be the so-called lactoperoxidase system (LP system). The use of this lactoperoxidase system, which has a broad range of applicability, is of increasing importance.

The lactoperoxidase/thiocyanate/hydrogen peroxide system is an antimicrobial system which is indigenous to the major body fluids such as raw milk, tears and saliva.

The properties of this system have been reviewed by Reiter and Harnülv (1984. J. Food Protect. 47 : 724-732) and Pruitt and Reiter (1985. In 'The lactoperoxidase system chemistry and biological significance' Eds. Pruitt, K.M. and Tenovuo, D. p. 144-178, New York , Marcel Dekker, Inc.).

Schematically, the lactoperoxidase system can be represented by a three-step process;

a) the hydrogen peroxide production step; the reaction of an oxidoreductase with an oxidizable substrate with the concommitant production of hydrogen peroxide,
b) the lactoperoxidase reaction step; in this step thiocyanate is converted to hypothiocyanate by reaction with hydrogen peroxide which reaction is catalyzed by lactoperoxidase,
c) the antimicrobial reaction; wherein hypothiocyanate inactivates the microorganisms.

FR-A-2237589 discloses the immobilization of enzymes suitable for the production of hydrogen peroxide such as glucose oxidase on a matrix through which the liquid to be treated (e.g. milk, thus a lactoperoxidase containing product) can flow.

In FR-A-2153443 (Example 17) a combination of invertase and glucose oxidase is immobilised and used in a reaction leading to the formation of hydrogen peroxide. In this Example the immobilisation is performed with gelatin.

In the method disclosed in EP-A-0397228 a glucose oxidase system is used as part of an antimicrobial composition for the preservation of cheese, in particular against contamination by Listeria; the glucose oxidase system is preferably isolated from the rest of the composition.

EP-A-0397227 is directed to a process for preparing an antimicrobial system containing at least a lactoperoxidase and optionally an oxido-reductase which are bound (anchored) to a particulate vector comprising a nucleus formed from a hydrophilic polysaccharide polymer, a first lipidic layer chemically bound to the nucleus and a second phospholipidic layer, the enzyme molecules being inserted in the second phospholipidic layer and/or in the first lipidic layer.

Instead of in situ productions of the hydrogen peroxide, hydrogen peroxide can also be slowly added to the mixture to be protected. Furthermore, it is possible to use soluble inorganic peroxides from which peroxide is gradually released. For practical reasons however it is better to generate the hydrogen peroxide in situ. Preferably the hydrogen peroxide is produced enzymatically. Enzymatic production of hydrogen peroxide can be performed by using a number of different enzyme/substrate combinations, e.g. a combination of an oxidoreductase with an oxidizable substrate, for example;

glucose/glucose oxidase,

L amino acid/L amino acid oxidase,
galactose/galactose oxidase,
lactose/β-galactosidase/glucose oxidase,
2-deoxyglucose/glucose oxidase.

It is possible to add both the substrate and/or the oxidoreductase to the system which is to be protected. It is also possible to use an enzyme which is already present in the substance for which protection is sought. For example, in milk the normally present xanthine oxidase can be used to generate hydrogen peroxide by addition of hypoxanthine as a substrate. This addition of substrate is necessary to activate the system.

Combinations of different substrates and enzymes are equally effective and may give even better results. For example, the combination of glucose oxidase with β-galactosidase can be employed in lactose containing substances, β-galactosidase causes splitting of lactose, yielding galactose and glucose, the latter carbohydrate is then further oxidized by glucose oxidase.

The antimicrobial activity of this system is due to the formation of hypothiocyanate in the following reaction;

$$H_2O_2 + SCN^- \xrightarrow{\text{lactoperoxidase}} H_2O + OSCN^-$$

↑
lactic acid bact./
leucocytes

Raw milk contains all components which are essential for this reaction; thiocyanate and lactoperoxidase are present as such and hydrogen peroxide is produced by lactic acid bacteria or leucocytes. The thiocyanate is converted into hypothiocyanous acid (HOSCN) which at the pH of milk exists mainly in the form of the hypothiocyanate ion.

It may be useful in order to prolong the activity of the LP system to add hydrogen peroxide and/or if appropriate one of the other components of the system if they are limiting to the reaction. The addition of hydrogen peroxide in turn is limited by the effects which this molecule has on the activity of the lactoperoxidase and other proteins.

The hypothiocyanate ion reacts specifically with free sulfhydryl groups, thereby inactivating several vital metabolic enzymes and membrane proteins.

The hypothiocyanate has a bacteriostatic or bactericidal effect on a wide range of microorganisms. Activities of hypothiocyanate have been reported for example against, Pseudomonads, Enterobacteriaceae, Listeria, Yersinia, Campylobacter and Salmonella.

Milk preservation is an important application of this system. More generally, dairy products can be conserved using this system.

Other applications of the system in a more or less isolated form have been described. US patent 4,320,116 describes the use of this system in animal feedstuff and a method for treating bacterial infections in the gastrointestinal tract of mammals. Canadian patent application 1167381-A describes the use of this system in tooth-paste.

In general this system has the advantage of being food-grade; a wide spectrum of possible applications can therefore be envisioned.

One of the major problems with the use of the LP system is its short working time. The literature pertaining to this system thus far only reports activity ranging from a few hours to a maximum of a few days. The major factors responsible for this short working time are;

a) the uncontrolled (and high) production rate of hydrogen peroxide and,
b) the high reactivity of the hydrogen peroxide.

Due to its short working time, the LP system provides only temporary protection against microbial infection. The protected substances are prone to renewed contamination and therefore the use of the LP system has been limited to date, to short term protection.

There is a need for a long term protection of food and feedstuffs by a naturally occurring antimicrobial system. The present invention provides such a system.

## Summmary of the invention

The present invention discloses methods and means for increasing the working time of the lactoperoxidase system. To achieve this increased working time the invention discloses immobilized components of the lactoperoxidase system

which provide for the sustained release of hydrogen peroxide. Sustained release of hydrogen peroxide makes steady and continuous production of hypothiocyanate possible.

The present invention discloses immobilized components of the LP system. A system is disclosed which gives hydrogen peroxide production for at least 42 days.

The invention further discloses the application of the immobilized lactoperoxidase system components in food and feed.

Description of the figures

Figures 1-15 show the effect of the LP system on the following microorganisms: Escherichia coli ATCC 11229, Salmonella typhimurium ATCC 13311, Bacillus cereus IAM 1229, Staphylococcus aureus ATCC 6538 and Listeria monocytogenes RIVM 3 at the indicated pH values.

Figure 16 shows the effect of the lactoperoxidase system on Camembert cheese infected with Listeria.

Figure 17 shows the production of d-gluconic acid in time from free glucose and from immobilized cornstarch.

Figure 18 shows the production of gluconic acid in time using immobilized cornstarch.

Detailed description of the invention

The present invention provides a composition for use in the sustained production of hydrogen peroxide comprising an oxidoreductase and a corresponding substrate in an immobilized form.

In its most general form, the present invention provides a method for obtaining sustained release of hydrogen peroxide by use of the composition. The hydrogen peroxide is slowly released and may be used for its antimicrobial activity as such. In order to exhibit antimicrobial activity, the hydrogen peroxide must be present in relatively large amounts. Hypothiocyanate is a much more powerful antimicrobial agent than hydrogen peroxide. Antimicrobial activity has been reported for hydrogen peroxide at a concentration of 5mM, whereas 0.02mM hydrogen peroxide has been reported to activate the LP system. The hydrogen peroxide is therefore preferably used to convert thiocyanate into hypothiocyanate with the use of lactoperoxidase or another peroxidase.

Any thiocyanate salt may in principle be used. Commonly alkali metal salts such as sodium or potassium thiocyanate are used.

To obtain slow release of hydrogen peroxide through the reaction of at least one oxidoreductase with its corresponding substrate the method is characterized in that at least the oxidoreductase is immobilized and the substrate (glucose, galactose or other substrate) is immobilized together with the oxidoreductase and/or generated by enzymatic or chemical reaction to make it available in usable form.

The composition of the present invention may contain any oxidoreductase. Preferably the oxidoreductase is selected from the group consisting of glucose oxidase, L amino acid oxidase, galactose oxidase, β-galactosidase/glucose oxidase, xanthine oxidase with a corresponding substrate. Combinations of oxidoreductases may advantageously be used in the present invention.

In the present invention the hydrogen peroxide is made available continuously and preferably at a steady-state level high enough to activate the lactoperoxidase system. To achieve this the substrate may for example be present in a slowly soluble form or it may be present in polymer form in which case the substrate molecule is only available in usable form after an enzymatic or chemical reaction.

It is also possible to couple the above hydrogen peroxide production step with another reaction step in which the substrate is generated, thereby indirectly regulating the hydrogen peroxide production rate by regulating the substrate release or production rate. An example of this is glucose which is obtained from cellulose by reaction with cellulase. Another example is the degradation of lactose using the combination of glucose oxidase with β-galactosidase. Yet another example is the use of starch as a substrate necessitating the prior release of glucose. After release of the substrate the oxidreductase reaction produces hydrogen peroxide. It has been found that immobilization of the components provides a prolonged glucose release rate.

The hydrogen peroxide thus obtained is preferably used for increasing the effective working time of the lactoperoxidase system. We focus our discussion on the lactoperoxidase system since this system is the system of choice for food applications. However, it is recognized that other enzymes can equally well be employed according to the present invention to generate hydrogen peroxide, for example horseradish peroxidase and chloroperoxidase.

The system of the present invention can schematically be represented as follows:

```
┌ - - - - - - - - - - - - - - - - - - - - - - ┐
  bound substrate  (cellulose, starch ..)
|                                             |
     (1) ↓ -  enzyme, chem. reaction
|                                             |
     substrate     (glucose, galactose...)
|                                             |
     (2) ↓ -  oxidoreductase
└ - - - - - - - - - - - - - - - - - - - - - - ┘
     hydrogen peroxide
     (3) ↓ - peroxidase
           - thiocyanate
     hypothiocyanate
```

Encircled is the part of the system wherein at least one of the components is immobilized.

To our knowledge it has not previously been attempted to use the LP system to obtain prolonged antimicrobial protection. Prolonged antimicrobial activity solves at the same time the problem of possible recontamination.

To date the LP system is generally used to treat the substance once, and subsequent reinfection is avoided by physical separation of the 'protected' substance from sources of contamination.

The present invention makes outgrowth of reinfecting microorganisms during a longer period impossible.

In case the substrate used for the enzymatic reaction is also a substrate for one of the microorganisms present in the compositions to be protected, or in case the substrate is a substrate for other infecting microorganisms, it is preferable to add the substrate in a non-metabolisable form. Different options for using a non-metabolisable substrate present themselves;

1) the substrate can be immobilized, for example in the form of cellulose or starch. By producing and subsequently oxidizing glucose in situ, at a reaction rate that prevents accumulation, the growth of microorganisms can be prevented,

2) alternatively a non-metabolisable substrate as such can be employed; 2-deoxyglucose is an example.

The system of the present invention can be employed against a wide range of organisms. As indicated above the hypothiocyanate, which is produced in the lactoperoxidase reaction step, has been found to be active against a wide range of microorganisms including both gram-positive and gram-negative bacteria and fungi.

Activities of the hypothiocyanate have been reported for example against, Pseudomonads, Enterobacteriaceae, Listeria, Yersinia, Campylobacter, Salmonella, Streptococcus, Lactobacillus, Bacteroides, Flavobacterium and Fusobacterium.

The spectrum of activity of the present system can be increased by combining the system with other antimicrobial agents. Where apart from general protection, protection against a specific microorganism is required, it may be useful to add a bacteriocin to the system as described. This addition may be done either before or after immobilisation. Suitable bacteriocins are known and include lantibiotics such as nisin.

The present invention exemplifies the use of the LP system against both gram-positive and gram-negative bacteria. Specifically it is shown that the system of the present invention is effective against the following microorganisms: Escherichia coli ATCC 11229, Salmonella typhimurium ATCC 13311, Bacillus cereus IAM 1229, Staphylococcus aureus ATCC 6538 and Listeria monocytogenes RIVM 3. Tests with these microorganism have been performed at different pH between 5 and 7. At all values the system works well, the preferred pH was 6.3.

The present invention focuses on step (2), the second step of the three-step process described above, that is the hydrogen peroxide production step. In order to obtain a steady-state level of hydrogen peroxide the amount produced should be kept constant. To achieve a constant hydrogen peroxide production level, the substrate for the peroxide formation reaction can be added in a controlled manner. Alternatively a limiting amount of enzyme, with an excess of substrate can be used.

The invention provides a method for controllably and slowly generating the hydrogen peroxide, this is achieved by the immobilisation of the enzymes and optionally the substrates. Immobilisation methods are known. Suitable methods make use of for example Calcium alginate, gelatin or carrageenan. If necessary the immobilized material can be reinforced by cross-linking agents.

In the present invention some of the possible compositions are exemplified. Avicel[tm] (cellulose) is immobilized together with cellulase and glucose oxidase in gelatin which is subsequently cross-linked with glutardialdehyde. In this system hydrogen peroxide is produced for at least 48 hours.

In another example cornstarch is immobilized together with $\alpha$-amylase, amyloglucosidase and glucose oxidase in a combination of gelatin and alginate, with subsequent cross-linking with glutardialdehyde. It is shown that this system is capable of releasing hydrogen peroxide for at least 42 days.

It is understood that the amounts of the components and the composition itself in the system will vary depending on the specific application. The coupling of the exemplified systems with lactoperoxidase/thiocyanate will increase the effectivity of the antimicrobial composition.

In liquid form minimal amounts of the LP components are as follows;

glucose oxidase (Gist-brocades) 0.8 mg/l,
lactoperoxidase (Biopole) 1 mg/l,
hydrogen peroxide 0.02 mM,
SCN 0.02mM.

In general the molar ratio between peroxide and thiocyanate is smaller than 4 and preferably it is 1-2. The lactoperoxidase is present in amounts varying from 1 - 200mg/l. Activities of the enzymes are as follows;
glucose oxidase, 36.000 units/g (pH=6, T=14°C), wherein 1U = 1µmol hydrogen peroxide /min,
lactoperoxidase 481.000 ABTS units/g (pH=6, T=25°C) (ABTS method, Childs et al. Biochem.J. (1975) 145 93-103).

Finally the present invention discloses a food product which when treated with $10^2$-$10^5$ microbial cells per g between 2-10 days after preparation and which is subsequently kept at normal growth conditions for the infecting microorganism does not give rise to outgrowth of this microorganism and wherein protection is due to sustained hydrogen peroxide production. Specifically, it is also shown that the LP system is effective against Listeria, when applied on cheese (Camembert).

Specific amounts of cells and growth conditions may of course vary depending on the nature of product and the microorganism which is employed.

When practising the invention, the substance to be protected is mixed with quantities of the reagents in such a way that the hydrogen peroxide will be generated in such an amount per unit time that a steady-state concentration is achieved.

The system as described, provides its antimicrobial protection for at least 10 days, preferably at least 20 days and more preferably up to 50 days.

Utility of the invention.

The system can be applied to food and feed conservation. With respect to this application, it can be used in a liquid, for example in (cheese-)milk, but it will be equally effective when applied to the surface of for example cheese. The system can also be applied as a longterm cleaning agent in specific applications. It is understood that the amounts of the components in the system will vary depending on the specific application.

The use of this system can be envisioned in the decontamination of carcases (bovine, fish, shrimps), surface treatment of food (cheese, butter), treatment of fresh vegetables, cosmetics, wound treatment, toothpaste, decontamination of machines (icemachines, milkshake machines) or more broadly equipment used in food processing in plants or in area wherein food is prepared in large amounts (hospitals, restaurants and the like), decontamination of udders, silage and in feedstuff.

Experimental

Hydrogen peroxide analysis

Measurement of the amount of hydrogen peroxide was performed by a modification of the method described Mottola et al. Anal. Chem. 42:410-411 (1970).

Briefly, in a 1 cm cuvet the following solutions were mixed;

50 µl sample containing hydrogen peroxide (0.2-1 mM)
200 µl leuco-crystal-violet (LCV) solution (0.5-1 mg/ml in 0.5% HCl)
1.6 ml Sodium acetate buffer (0.5 mM pH 4.5)
100 µl lactoperoxidase (2 mg/ml) or HRP (Horse Radish Peroxidase)

In the presence of thiocyanate, lactoperoxidase can not be used to obtain accurate measurements in this assay. However, under these conditions horse radish peroxidase works well.

Color development was followed at 596nm.

Composition of media

Minimal medium contained the following substances per liter;
$K_2HPO_4$, 14g; $KH_2PO_4$, 6g; $(NH_4)_2SO_4$, 2g; Trisodiumcitrate. $2H_20$, 1g; $MgSO_4$. 7 $H_2O$, 0.2mg; $MnSO_4$. $2H_2O$, 5g; L-glutamic acid, 2g; NaOH, 0.8g; 50ml 10% Casamino acid solution (Difco), 20ml 50% glucose solution and 10ml Vitamin solution.
Vitamin solution contained per liter;
2mg biotin; 2mg folic acid; 10mg pyridoxine HCl B6; 5mg thiamine HCl B1; 5mg riboflavin B2, 5mg nicotinic acid; 0.1mg vitamin B12; 5mg p-aminobenzoic acid; 5mg DL Calciumpenthotenate.
Cheese milk medium (CM medium) contained per liter;
15g Caseinehydrolysate, 3g tri-sodiumcitrate, 3g lactose, 3.5g lactate, 5g tryptose and 50mM phosphate buffer (pH 5, 6 or 7).
After sterilisation glucose was added.

Examples

Example 1

Activity of the lactoperoxidase system against specific microorganisms

The activity of the lactoperoxidase system against five different microorganisms was tested using glucose oxidase/glucose to generate hydrogen peroxide.

The microorganisms used were the following:

| | |
|---|---|
| Gram-negative: | Escherichia coli ATCC 11229 |
| | Salmonella typhimurium ATCC 13311 |
| Gram-positive: | Bacillus cereus IAM 1229 |
| | Staphylococcus aureus ATCC 6538 |
| | Listeria monocytogenes RIVM 3 |

E.coli, S.tyhimurium, B.cereus and S.aureus were incubated at the desired pH in minimal medium.

L.monocytogenes was incubated in cheese milk medium. After overnight culture the cells were used to inoculate the main culture to a density of $10^3$-$10^5$ cells/ml. The incubation temperature was 37°C. The pH was 5.2, 6.3 or 7.2 (for L.monocytogenes; 5.0, 6.0 and 7.0). To these cultures the given substances were added to the indicated final concentrations:

- $SCN^-$ 100mg/l (sodium salt, Merck);
- lactoperoxidase 20mg/l (Biopole);
- glucose-oxidase 1.5 mg/l (Gist-brocades);
- glucose 10 g/l (BDH).

The control cultures contained the same substances without glucose-oxidase.

The number of viable cells was followed in time and determined by plating several dilutions on BHI plates.

The hydrogen peroxide concentration was monitored during these experiments using the method outlined in Experimental. It could be concluded that using the concentrations mentioned above the hydrogen peroxide was never present in an amount sufficient to have any microbial effect as such. Thus, the antimicrobial effects described could completely be attributed to the hypothiocyanate.
Results are shown in Figures 1-15.

E.coli (Fig. 1-3)

| | |
|---|---|
| pH=7.2 | - cells killed between 8 and 24 hours |
| | - control continues to grow after a lag phase of 4 hours |
| pH=6.3 | - cells killed after 4 hours |
| | - control continues to grow after 4 hours |

pH=5.2 - cells killed after 2 hours
- control continues to grow after 6 hours

<u>S.typhimurium</u> (Fig. 4-6)

pH=7.2 - cells killed between 6 and 24 hours
- control continues to grow after 4 hours
pH=6.3 - cells killed within 2 hours
- control continues to grow after 6 hours
pH=5.2 - cells killed after 4 hours
- control continues to grow after 4 hours

<u>S.aureus (Fig. 7-9)</u>

pH=7.2 - cells not completely killed
- control continues to grow after 8-24 hours
pH=6.3 - cells killed within 4 hours
- control as with pH=7.2
pH=5.2 - cells killed within 6 hours
- control as with pH=7.2

<u>B.cereus</u> (Fig. 10-12)

pH=7.2 - cells killed within 2 hours
- control continues to grow after 2 hours
pH=6.3 - cells killed as with pH=7.2
- control continues to grow after 4 hours
pH=5.2 - cells killed between 6 and 24 hours
- control continues to grow after 6 hours

<u>L. monocytogenes</u> (Fig. 13-15)

pH=7.0 - cells killed between 5 and 24 hours
- control continues to grow after 5 hours
pH=6.0 - cells killed between 3 and 24 hours
- control continues to grow after 2 hours
pH=5.0 - cells killed between 3 and 24 hours
- control does not grow at this pH.

It can be concluded that all tested microorganisms are adequately killed under the given experimental conditions, except for <u>S.aureus</u> at pH=7.2.
Overall pH=6.3 is the optimal pH.

<u>Example 2</u>

<u>Sustained release of hydrogen peroxide</u>

5g of Avicel$^{(tm)}$ (a crystalline polymer constisting of cellulose, Serva) was suspended in 45 ml of an aqueous solution of gelatin, at 30°C. Subsequently 20mg cellulase (Gist-brocades, Maxazym$^{tm}$ CL 2000) and 25mg glucose oxidase (Gist-brocades) were added. This gelatin-polymer-enzyme suspension was added to 100ml of a stirred cornoil (Brocacef) solution at 30°C. The water in oil suspension was cooled to 10°C. The particles are cross-linked by slowly adding (in 60 min) 1.03 g glutardialdehyde (Merck) in 8.25 ml water.

0.05g Tween 80 in 5 ml water was added and stirring was continued for 5 min.

Subsequently the particles were separated from the oil phase by addition of 1000ml water and the particles were washed twice with the same amount of water. The particles were stable and insoluble in water.

The release of hydrogen peroxide was followed in time using the method described in the Experimental section. The experiment was performed by incubating 5 g of the particles in 50ml buffer (pH=5.0) in a stirred reactor vessel at room temperature.

The results are shown in Table 1. It can be concluded that hydrogen peroxide production is sustained and constant.

Table 1

| Generation of hydrogen peroxide | |
|---|---|
| time (h) | µmol $H_2O_2$ /h/g particles |
| 24 | 0.020 0.021 |
| 48 | 0.022 0.019 |

Example 3

Effect of the lactoperoxidase system on Camembert cheese infected with Listeria monocytogenes

Camembert cheese frozen 1 day after production was kept frozen at -50°C. Freezing and thawing had no visible effect on the cheese flora. Cheeses were put in refrigerator boxes with a volume of 1 liter and the relative humidity was kept at 95% using a glycerol/water mixture.

The boxes were incubated at 14°C. After 5 days cheeses were treated with Listeria monocytogenes DSM 20600 at 100 cells per gram cheese (in 0.5ml). After 4 hours the cheeses were treated at one side with 0.6ml of LPS solution (100mM glucose (BDH), 20mM NaSCN (Merck), 200mg/l lactoperoxidase (Biopole) and 50mg/l glucose oxidase (Gist-brocades)). Control cheeses were treated with milli-Q water.

The number of Listeria were counted at t=0 and after 1, 2 and 5 days in duplo. Counting was performed by diluting 17 g of cheese two times in 2% tri-sodiumcitrate. After homogenisation in a Stomacher the suspension was diluted in a physiological salt solution.

0.1 ml of different dilutions were brought onto Palcam plates (Merck). Plates were grown at 30°C during one day and colonies were counted.

The result is shown in Figure 16 and it can be concluded that the LPS system works well under application conditions.

Example 4

Use of immobilized cornstarch as a glucose source I

A suspension consisting of 10% (w/w) cornstarch in 8% (w/w) gelatin and 1% (w/w) alginate was prepared. The mixture was kept at 30°C and 0.05% α-amylase (Gist-brocades, Maxamyl$^{tm}$, 6300 TAU/g), 0.05% amyloglucosidase (Gist-brocades, Amigase$^{tm}$ TS, 25000 AGI/ml) and 0.05% glucose oxidase (Gist-brocades) (all on w/w basis) were added.

Subsequently the suspension was poured into two volumes of cornoil containing 1% (w/w) Span 80. The mixture was heavily stirred using a turbin rotor. After 5 minutes the temperature was lowered to 15°C and after coagulation 8.25 ml cross-link mixture was added per 50 g of formulation.
Cross-link mixture consisted of 88.5% $CaCl_2$. $2H_2O$ in ethanol (40 g per 100ml ethanol) and 11.5% glutardialdehyde (25% w/w).
After 60 minutes the immobilisation product-oil emulsion was stirred in an excess water for 5 minutes and the oil was decanted. The immobilisation product was subsequently washed twice with an excess of water and finally isolated by fractionated sieving.

In order to follow the hydrogen peroxide production rate production of gluconic acid was measured. Gluconate is a product of the hydrogen peroxide forming reaction:

$$\text{glucose} + O_2 \rightarrow H_2O_2 + \text{gluconate}$$

Two open, stirred 100ml reaction vessels were used to follow the D-gluconic acid production at room temperature.
Vessel one contained 2.5 g of immobilisation product in 25 ml 0.1 M sodium acetate buffer, pH 5.
Vessel two contained 0.277 g glucose and 1.38 g glucose oxidase in 25 ml 0.1 M sodium acetate buffer, pH 5.
D-gluconic acid was measured using an enzymatic test kit from Boehringer Mannheim (cat. no. 428.191).

The results are presented in Figure 17. It can be seen that without immobilisation and use of free glucose the D-gluconic acid production stops after about 50 hours whereas D-gluconic acid production and hence hydrogen peroxide production continues for more than 200 hours when immobilized starch is used as glucose source.

The maximum amount of gluconic acid which could be obtained from the amount of starch used in this experiment was 60 mM.

Example 5

Use of immobilized cornstarch as a glucose source II

50 g of cornstarch was suspended in 200 ml water and heated to 85°C. The slurry was kept at this temperature for 15 minutes with continues stirring. A solution of 50 g gelatine in 200 ml was added. After cooling the suspension to about 40°C, 125 mg amyloglucosidase (Amigase$^{tm}$ TS), 250 mg glucose oxidase and 1250 mg lactoperoxidase were added. Thereafter 6 ml glutardialdehyde (25% w/w) was added with continuous stirring. The gel was homogenized with a blender. After homogenisation 2 l, 0.2 M sodium acetate containing 0.7% glutardialdehyde was added. The mixture was stirred for 15 minutes at 15°C. The product was sieved and washed twice with a tenfold volume of water. The product was subsequently dried in a fluid bed dryer at 39°C to 94% (w/w) dry weight.
Finally the dried particles were milled in a high speed hammer mill to a particle size of approximately 20 microns.

150 mg of the dried particles (20 microns) was suspended in 149 ml 0.1 M sodiumphosphate buffer pH=7.5, containing 4.5% (w/v) NaCl and 1.0 ml 400 mM NaSCN.

Incubation was in a shaking waterbath at 7°C with such a speed that the particles were kept in constant motion and that aeration was assured. Gluconic acid production was followed in time using the previously mentioned Boehringer test kit.
Figure 18 shows the results. Under the given conditions hydrogen peroxide can be generated for at least 42 days at a constant rate.

The maximum amount of gluconic acid which could be obtained from the amount of starch used in this experiment was 3 mM.

## Claims

1. A method for obtaining sustained release of hydrogen peroxide through the reaction of at least one oxidoreductase with its corresponding substrate characterized in that at least the oxidoreductase and the substrate is immobilized together with the oxidoreductase and/or generated by enzymatic or chemical reaction to make it available in usable form.

2. A method according to claim 1, characterized in that the immobilization is performed with gelatin, alginate, carrageenan.

3. A method according to claim 1 or 2 wherein the oxidoreductase is selected from the group consisting of glucose oxidase, L amino acid oxidase, galactose oxidase, β-galactosidase/glucose oxidase and xanthine oxidase.

4. A method according to any one of the previous claims wherein a combination of at least two oxidoreductases is employed.

5. A method of any one of the previous claims wherein the hydrogen peroxide is used to convert thiocyanate into hypothiocyanate in the presence of a peroxidase.

6. A method of claim 5 wherein the peroxidase is lactoperoxidase.

7. A method according to claim 5 or 6 whereby the hypothiocyanate is used in the preservation of food or feed.

8. A method according to claim 7 whereby the hypothiocyanate is used in the conservation of cheese.

9. A method according to claim 7 whereby the hypothiocyanate is used against gram-positive or gram-negative bacteria or fungi.

10. A method according to claim 9 whereby the hypothiocyanate is used against the following microorganisms: Escherichia coli, Salmonella typhimurium, Bacillus cereus, Staphylococcus aureus or Listeria monocytogenes.

11. A composition capable of producing hydrogen peroxide containing an oxidoreductase in an immobilized form and a corresponding substrate in an immobilized form regulating the release or production of the substrate to provide a sustained release of hydrogen peroxide.

12. A composition according to claim 11 characterized in that the oxidoreductase is selected from the group consisting of glucose oxidase, L amino acid oxidase, galactose oxidase and β-galactosidase/glucose oxidase and xanthine oxidase.

13. A composition according to claim 11 containing starch, amyloglucosidase, α-amylase and an oxidoreductase all in an immobilized form.

14. A composition according to claim 13, wherein the components are immobilized in alginate, gelatine or carrageenan.

15. A food product containing a composition according to any one of claims 11 to 14.

16. A food product according to claim 15 characterized in that the food product is a cheese.

**Patentansprüche**

1. Verfahren zur Erzielung einer verzögerten Freisetzung von Wasserstoffperoxid durch Reaktion von mindestens einer Oxidoreductase mit ihrem entsprechenden Substrat, dadurch gekennzeichnet, dass mindestens die Oxidoreductase immobilisiert wird und das Substrat zusammen mit der Oxidoreductase immobilisiert und/oder durch enzymatische oder chemische Reaktion erzeugt wird, damit es in brauchbarer Form zugänglich gemacht wird.

2. Verfahren nach Anspruch 1, dadurch gekennzeichnet, dass die Immobilisierung mit Gelatine, einem Alginat oder Carrageen durchgeführt wird.

3. Verfahren nach Anspruch 1 oder 2, in welchem die Oxidoreductase aus der Gruppe ausgewählt wird, welche aus der Glukose-oxidase, der L-Aminosäure-oxidase, der Galactose-oxidase, der β-Galactosidase/Glukose-oxidase und der Xanthin-oxidase besteht.

4. Verfahren nach einem der vorhergehenden Ansprüche, in welchem eine Kombination von mindestens zwei Oxidoreductasen verwendet wird.

5. Verfahren nach einem der vorhergehenden Ansprüche, in welchem das Wasserstoffperoxid zur Umwandlung von Thiocyanat in Hypothiocyanat in Gegenwart einer Peroxidase verwendet wird.

6. Verfahren nach Anspruch 5, in welchem die Peroxidase die Lactoperoxidase ist.

7. Verfahren nach Anspruch 5 oder 6, wobei das Hypothiocyanat zur Konservierung von Nahrung oder Futter verwendet wird.

8. Verfahren nach Anspruch 7, wobei das Hypothiocyanat zur Konservierung von Käse verwendet wird.

9. Verfahren nach Anspruch 7, wobei das Hypothiocyanat gegen gram-positive oder gram-negative Bakterien oder Pilze verwendet wird.

10. Verfahren nach Anspruch 9, wobei das Hypothiocyanat gegen die folgenden Mikroorganismen verwendet wird: Escherichia coli, Salmonella typhimurium, Bacillus cereus, Staphylococcus aureus oder Listeria monocytogenes.

11. Zubereitung die fähig ist, Wasserstoffperoxid herzustellen, enthaltend eine Oxidoreductase in immobilisierter Form und ein entsprechendes Substrat in immobilisierter Form, welche die Freisetzung oder die Erzeugung des Substrates reguliert, um eine verzögerte Freisetzung von Wasserstoffperoxid zu gewährleisten.

12. Zubereitung nach Anspruch 11, dadurch gekennzeichnet, dass die Oxidoreductase aus der Gruppe ausgewählt ist, welche aus der Glukose-oxidase, der L-Aminosäure-oxidase, der Galactose-oxidase, der β-Galactosidase/Glukose-oxidase und der Xanthin-oxidase besteht.

13. Zubereitung nach Anspruch 11, welche Stärke, Amyloglucosidase, α-Amylase und eine Oxidoreductase, alle in immobilisierter Form, enthält.

14. Zubereitung nach Anspruch 13, in welcher die Komponente in einem Alginat, in Gelatine oder in Carrageen immobilisiert sind.

15. Nahrungsmittel, enthaltend eine Zubereitung nach einem der Ansprüche 11 bis 14.

16. Nahrungsmittel nach Anspruch 15, dadurch gekennzeichnet, dass es ein Käse ist.

## Revendications

1. Procédé pour l'obtention d'une libération retardée de peroxyde d'hydrogène, par la réaction d'au moins une oxydoréductase avec son substrat correspondant, caractérisé en ce que la ou les oxydoréductases sont immobilisées et en ce que le substrat est immobilisé avec l'oxydoréductase et/ou généré par une réaction enzymatique ou chimique pour le rendre disponible sous une forme utilisable.

2. Procédé suivant la revendication 1, caractérisé en ce que l'immobilisation est réalisée au moyen de gélatine, d'alginate, de carragène.

3. Procédé suivant la revendication 1 ou 2, dans lequel l'oxydoréductase est choisie parmi le groupe consistant en glucose oxydase, L-acide aminé oxydase, galactose oxydase, β-galactosidase/glucose oxydase et xanthine oxydase.

4. Procédé suivant l'une quelconque des revendications précédentes, dans lequel on utilise une combinaison d'au moins deux oxydoréductases.

5. Procédé suivant l'une quelconque des revendications précédentes, dans lequel le peroxyde d'hydrogène est utilisé pour convertir le thiocyanate en hypothiocyanate, en présence d'une peroxydase.

6. Procédé suivant la revendication 5, dans lequel la peroxydase est la lactoperoxydase.

7. Procédé suivant la revendication 5 ou 6, dans lequel l'hypothiocyanate est utilisé dans la conservation de nourriture ou d'aliment.

8. Procédé suivant la revendication 7, dans lequel l'hypothiocyanate est utilisé dans la conservation du fromage.

9. Procédé suivant la revendication 7, dans lequel l'hypothiocyanate est utilisé contre des bactéries gram-positives ou gram-négatives ou des champignons.

10. Procédé suivant la revendication 9, dans lequel l'hypothiocyanate est utilisé contre les microorganismes suivants : Escherichia coli, Salmonella typhimurium, Bacillus cereus, Staphylococcus aureus ou Listeria monocytogenes.

11. Composition capable de produire du peroxyde d'hydrogène, contenant une oxydoréductase sous une forme immobilisée, et un substrat correspondant sous une forme immobilisée, régulant la libération ou la production du substrat pour fournir une libération étalée de peroxyde d'hydrogène.

12. Composition suivant la revendication 11, caractérisée en ce que l'oxydoréductase est choisie à partir du groupe consistant en glucose oxydase, L-Acide aminé oxydase, galactose oxydase, β-galactosidase/glucose oxydase et xanthine oxydase.

13. Composition suivant la revendication 11, contenant de l'amidon, l'amyloglucosidase, l'α-amylase et une oxydoréductase, tous sous forme immobilisée.

14. Composition suivant la revendication 13, dans laquelle les composants sont immobilisés sur de l'alginate, de la gélatine ou du carragène.

15. Produit alimentaire contenant une composition suivant l'une quelconque des revendications 11 à 14.

16. Produit alimentaire suivant la revendication 15, caractérisé en ce que le produit alimentaire est un fromage.

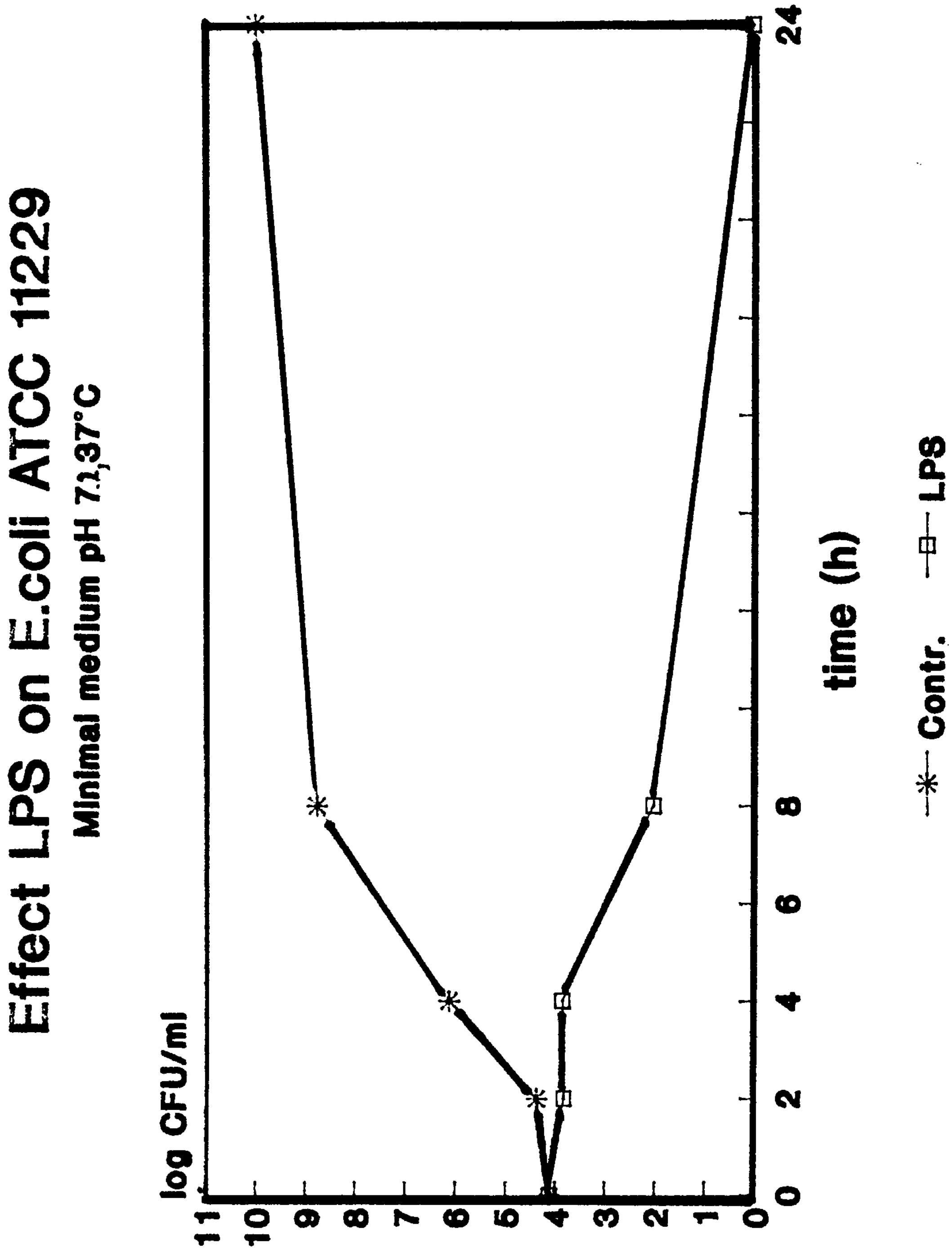
Effect LPS on E.coli ATCC 11229
Minimal medium pH 7.1,37°C
log CFU/ml
0
1
2
3
4
5
6
7
8
9
10
11
0
2
4
6
8
24
time (h)
Contr.
LPS

Figure 1

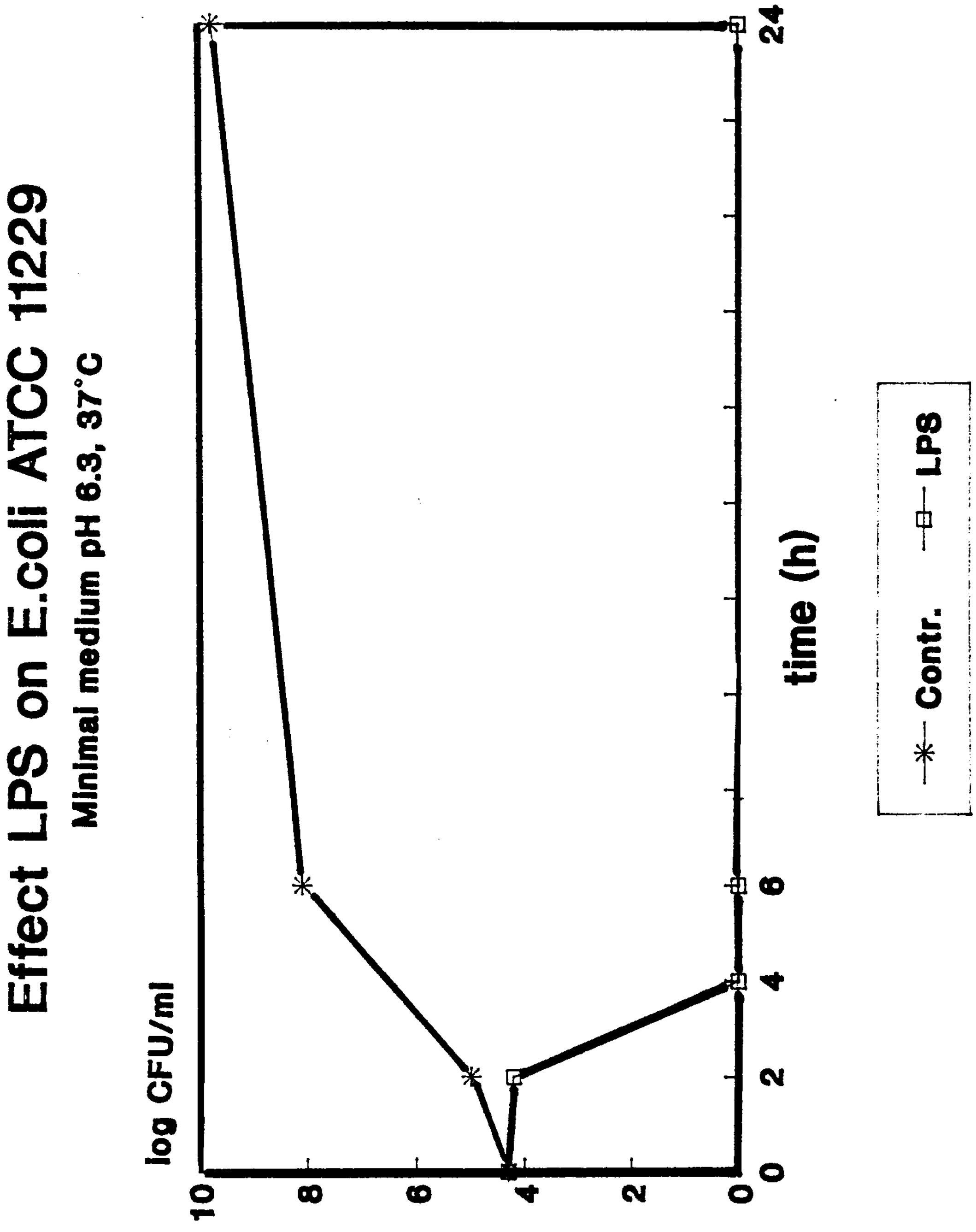

Effect LPS on E.coli ATCC 11229
Minimal medium pH 6.3, 37°C
log CFU/ml
10
8
6
4
2
0
0
2
4
6
24
time (h)
Contr.
LPS


Figure 2

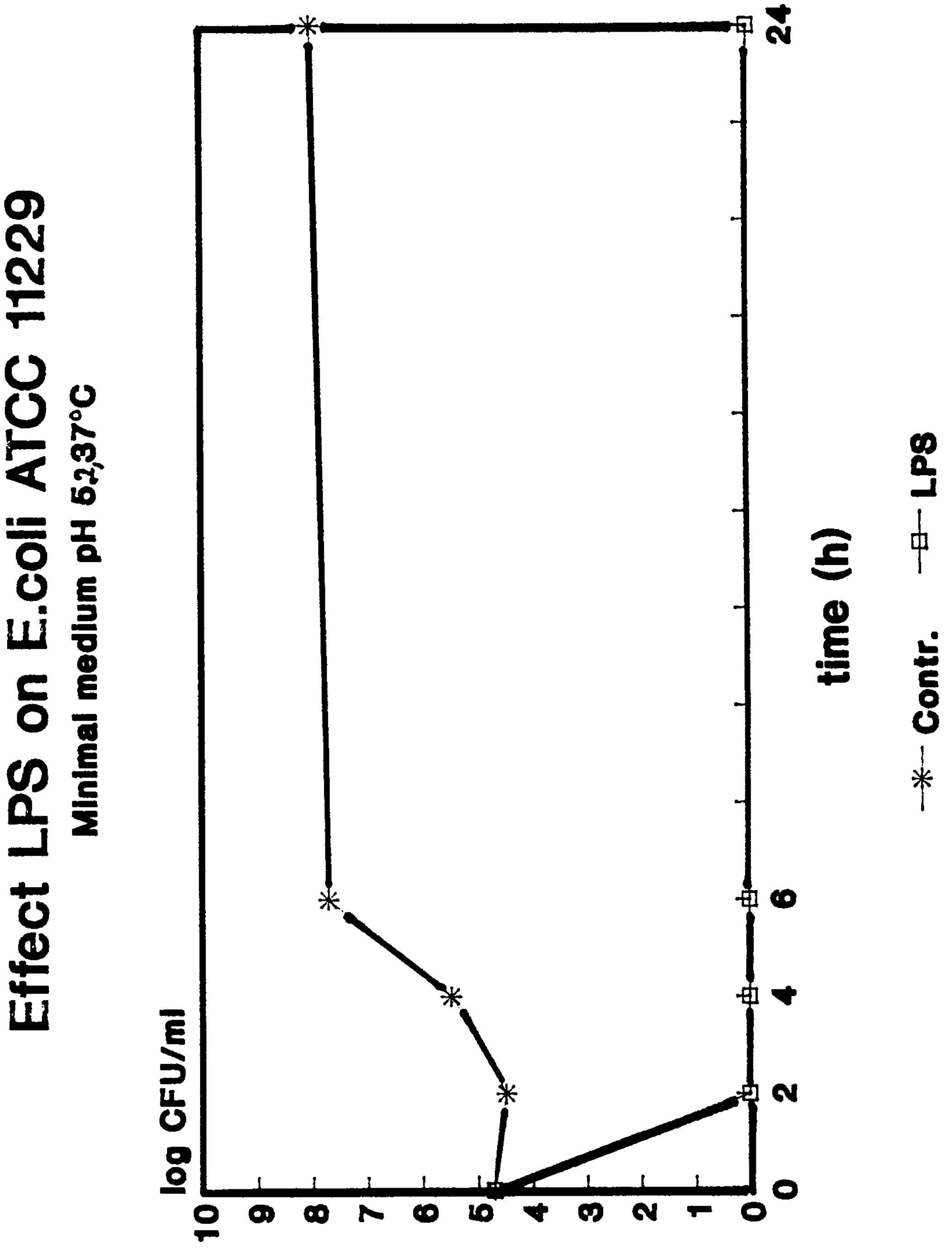
Effect LPS on E.coli ATCC 11229
Minimal medium pH 5,2,37°C
log CFU/ml
10
9
8
7
6
5
4
3
2
1
0
0
2
4
6
24
time (h)
Contr.
LPS

Figure 3

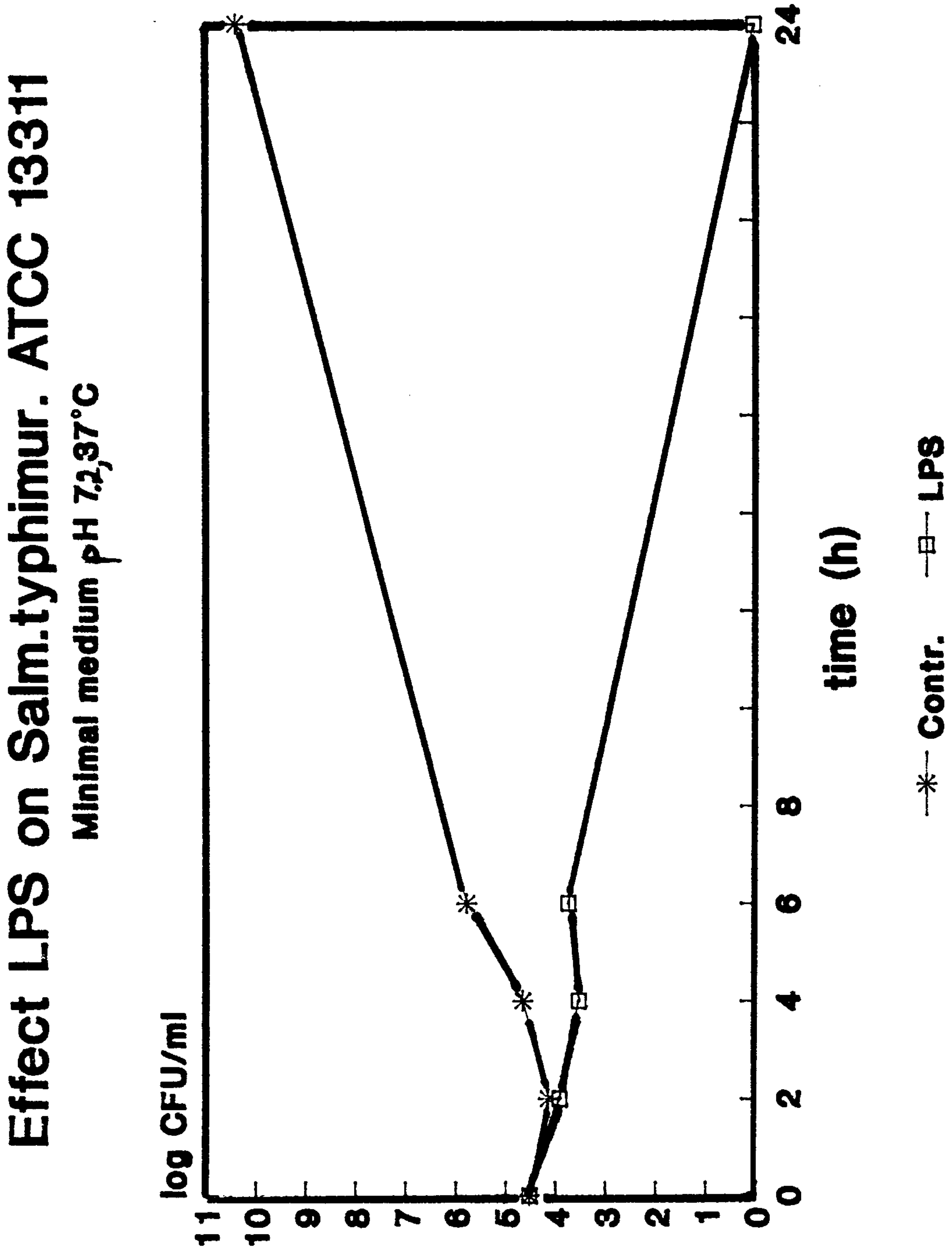

Effect LPS on Salm.typhimur. ATCC 13311
Minimal medium pH 7,2, 37°C
log CFU/ml
11
10
9
8
7
6
5
4
3
2
1
0
0
2
4
6
8
24
time (h)
Contr.
LPS


Figure 4

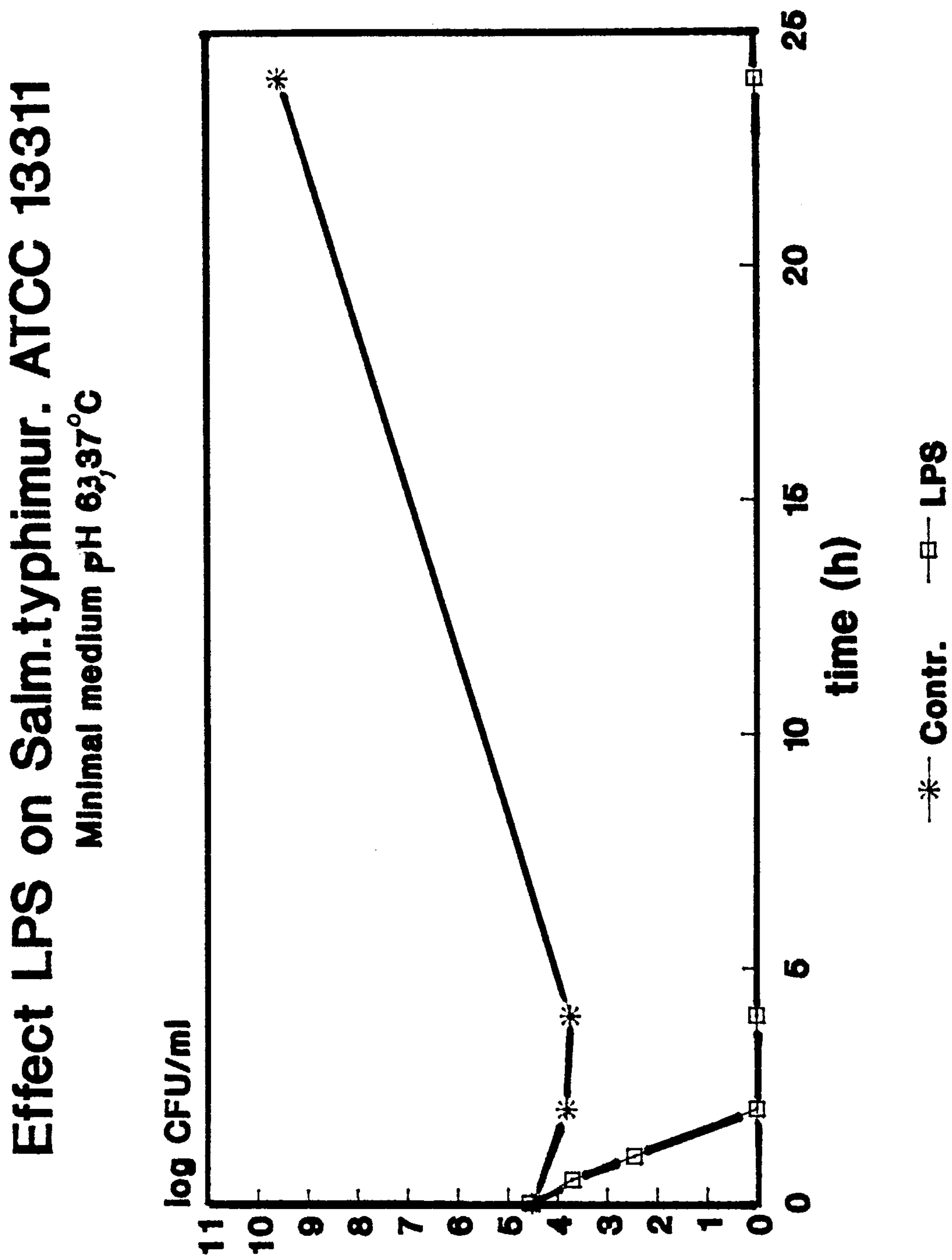

Effect LPS on Salm.typhimur. ATCC 13311
Minimal medium pH 6,3, 37°C
log CFU/ml
11 10 9 8 7 6 5 4 3 2 1 0
0 5 10 15 20 25
time (h)
Contr.
LPS


Figure 5

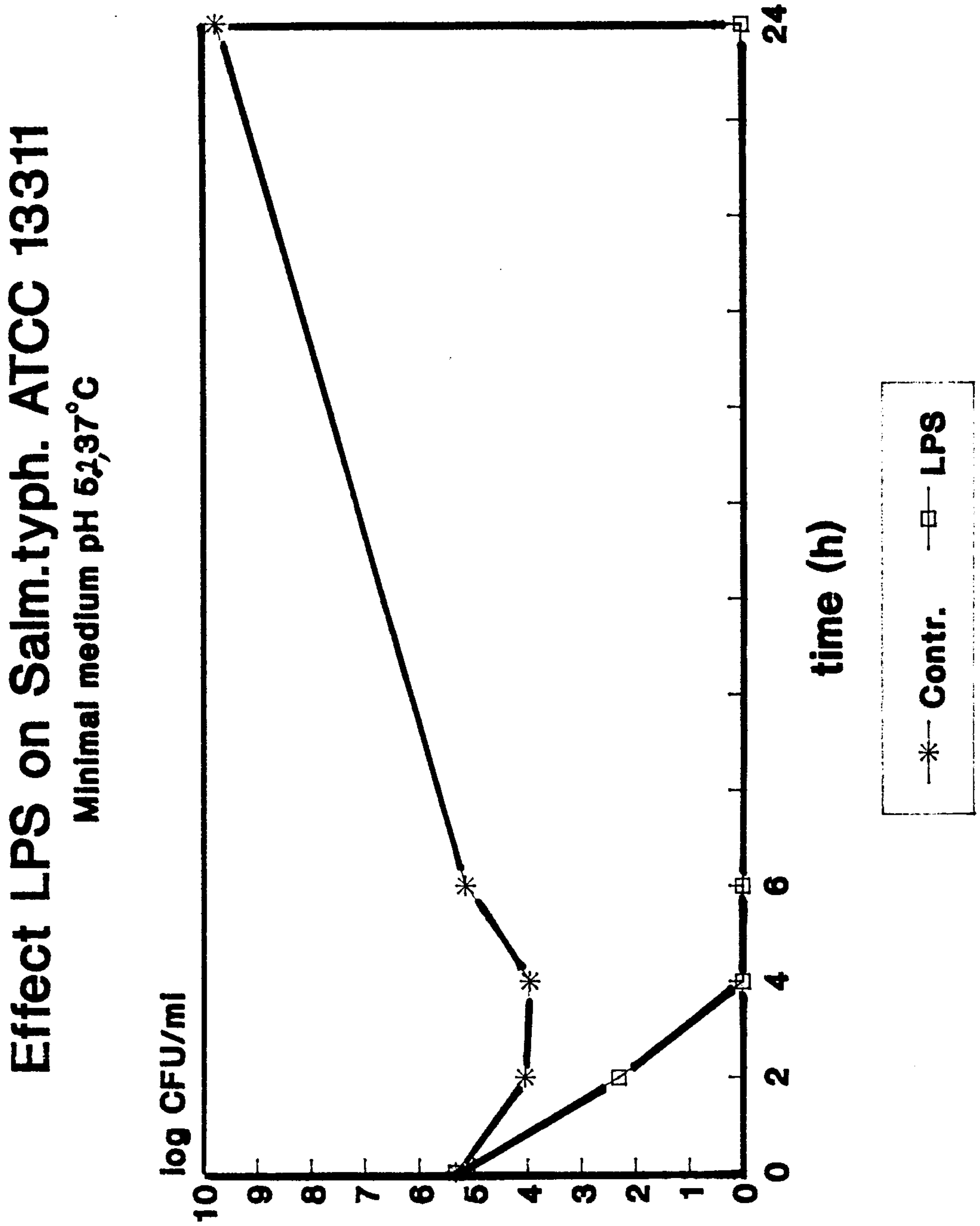
Effect LPS on Salm.typh. ATCC 13311
Minimal medium pH 5,2 37°C
log CFU/ml
10
9
8
7
6
5
4
3
2
1
0
0
2
4
6
24
time (h)
Contr.
LPS

Figure 6

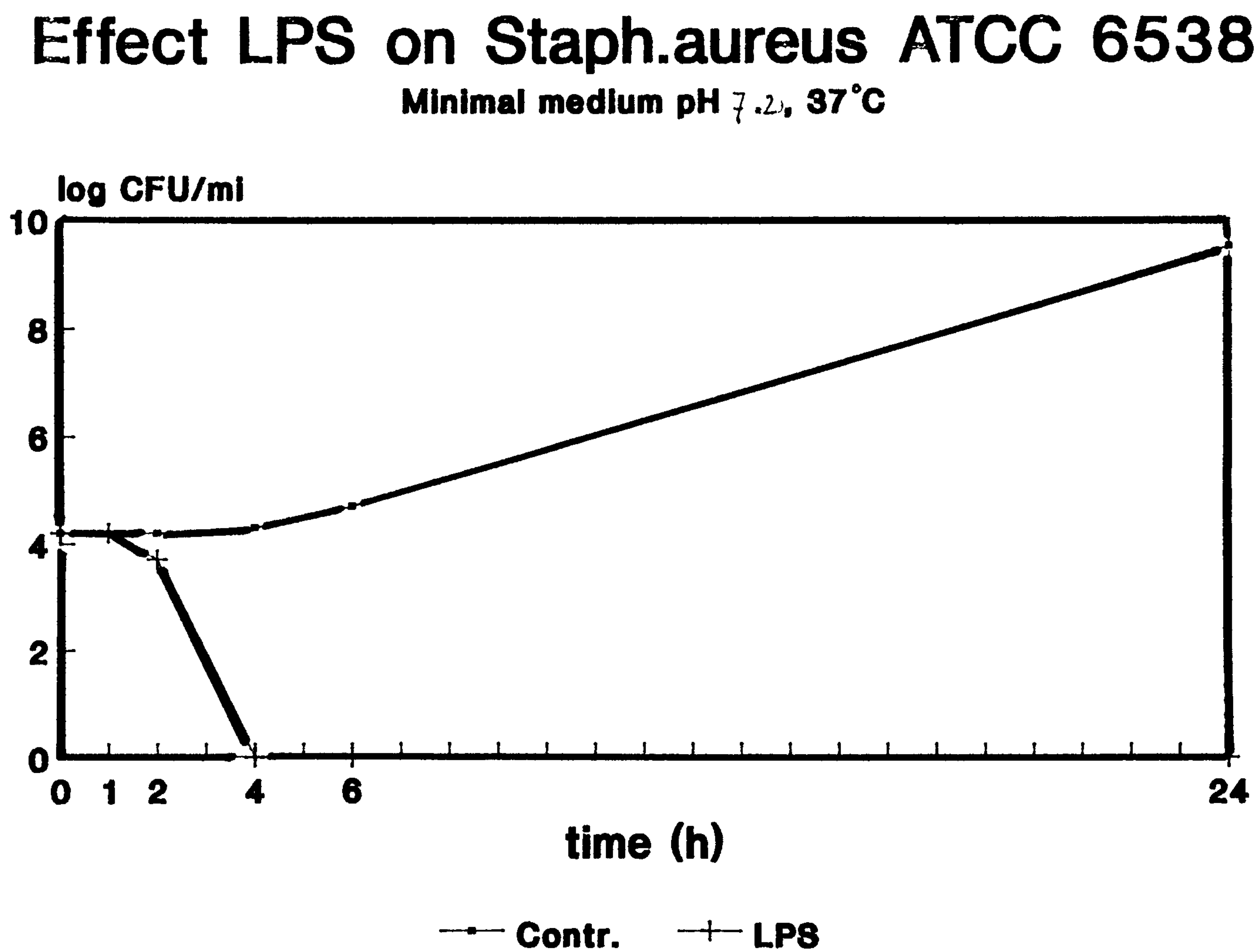
Effect LPS on Staph.aureus ATCC 6538
Minimal medium pH 7.2, 37°C
log CFU/ml
10
8
6
4
2
0
0
1
2
4
6
24
time (h)
Contr.
LPS

Figure 7

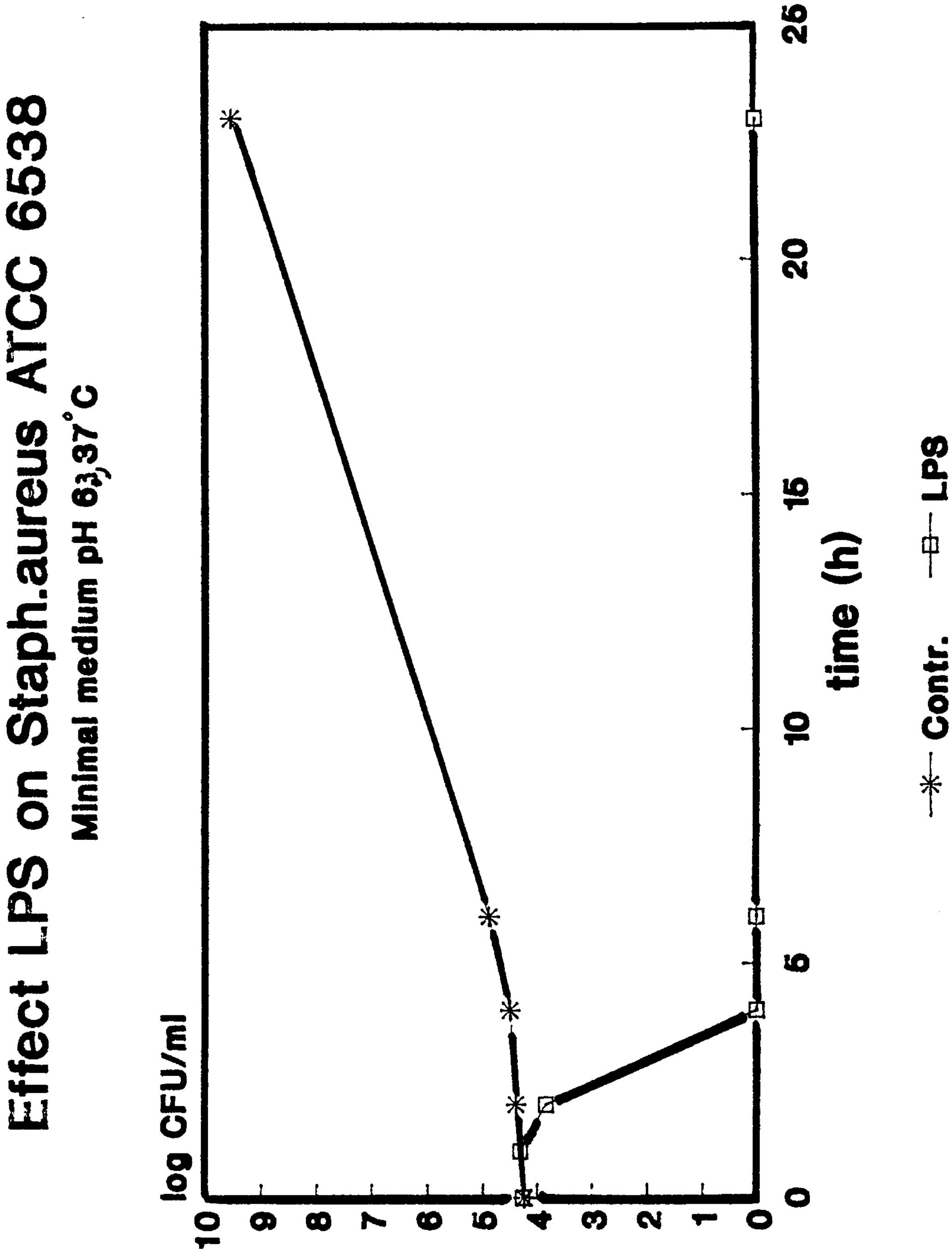
Effect LPS on Staph.aureus ATCC 6538
Minimal medium pH 6,37°C
log CFU/ml
10
9
8
7
6
5
4
3
2
1
0
0
5
10
15
20
25
time (h)
Contr.
LPS

Figure 8

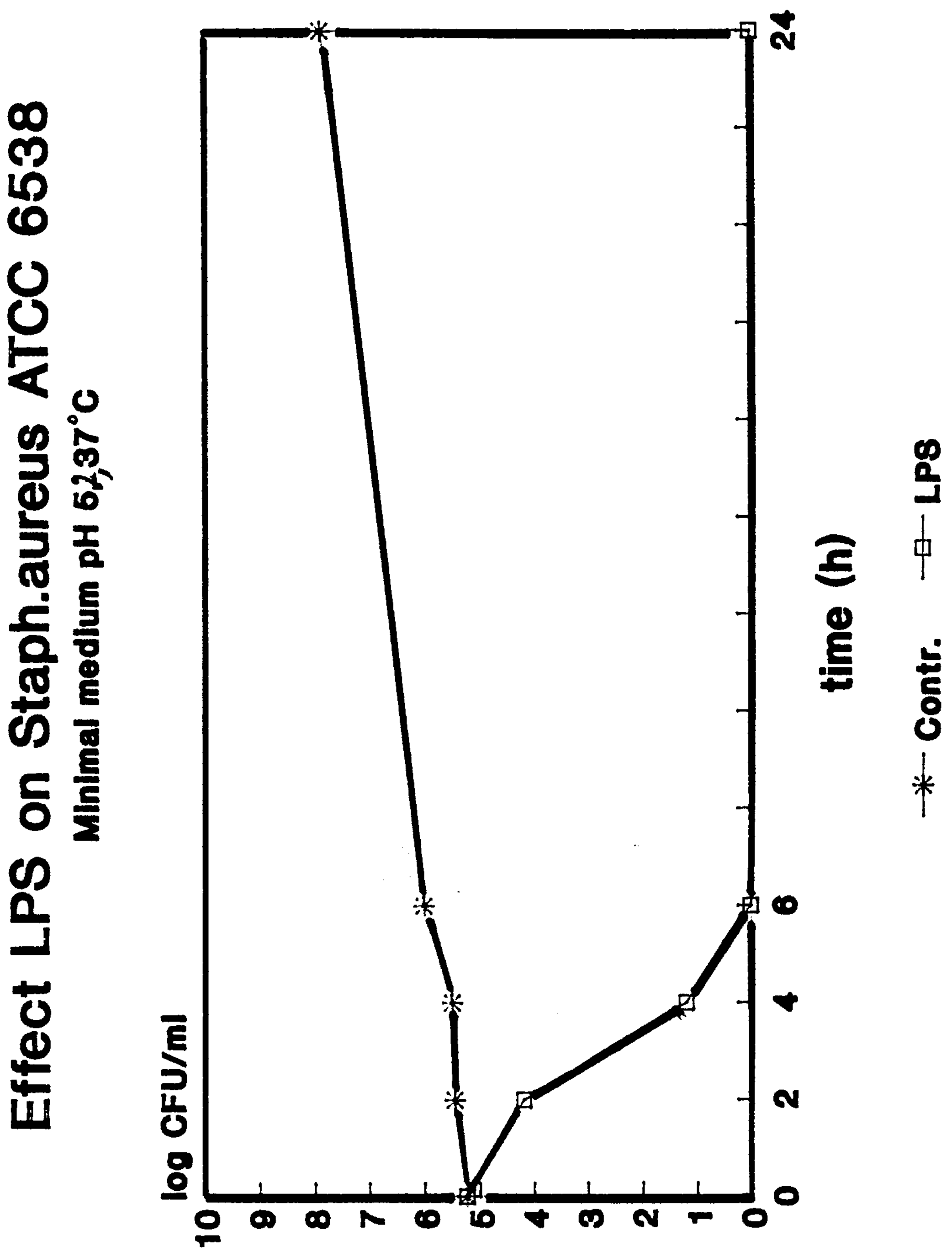

Effect LPS on Staph.aureus ATCC 6538
Minimal medium pH 5,2, 37°C
log CFU/ml
10
9
8
7
6
5
4
3
2
1
0
0
2
4
6
24
time (h)
Contr.
LPS


Figure 9

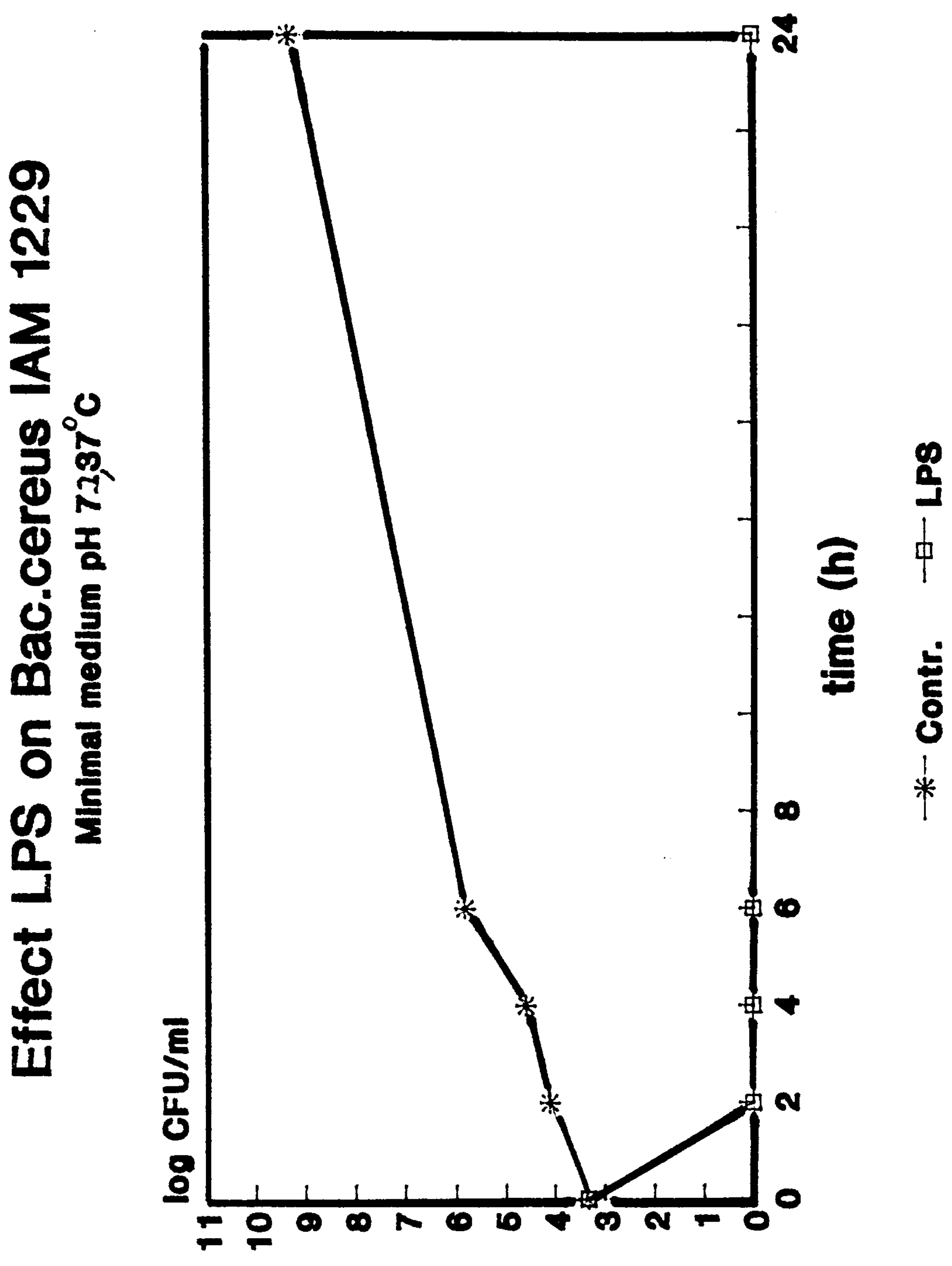
Effect LPS on Bac.cereus IAM 1229
Minimal medium pH 7,37°C
log CFU/ml
11
10
9
8
7
6
5
4
3
2
1
0
0
2
4
6
8
24
time (h)
Contr.
LPS

Figure 10

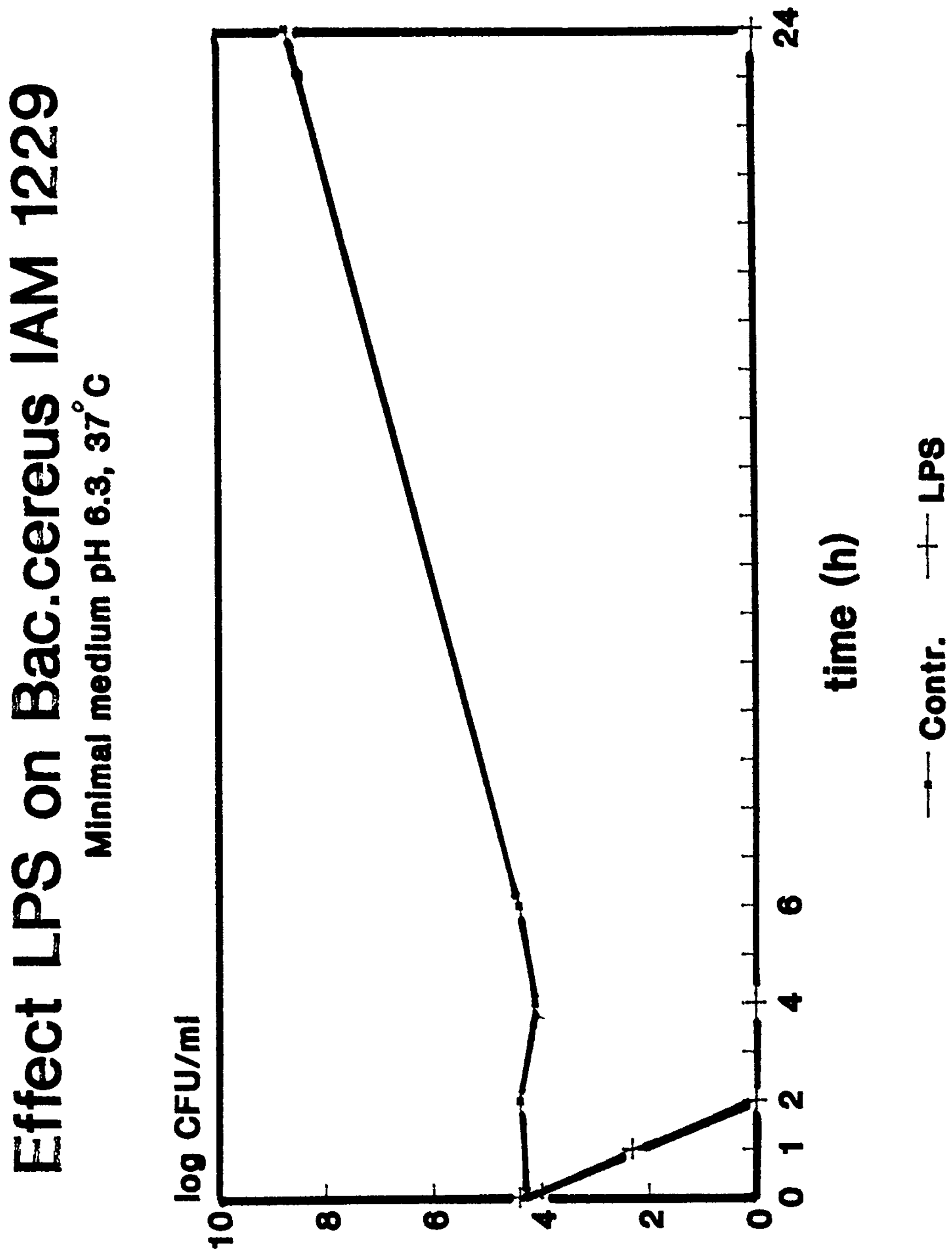
Effect LPS on Bac.cereus IAM 1229
Minimal medium pH 6.3, 37°C
log CFU/ml
10
8
6
4
2
0
0
1
2
4
6
24
time (h)
Contr.
LPS

Figure 11

## Effect LPS on Bac.cereus IAM 1229

**Minimal medium pH 5.1, 37°C**

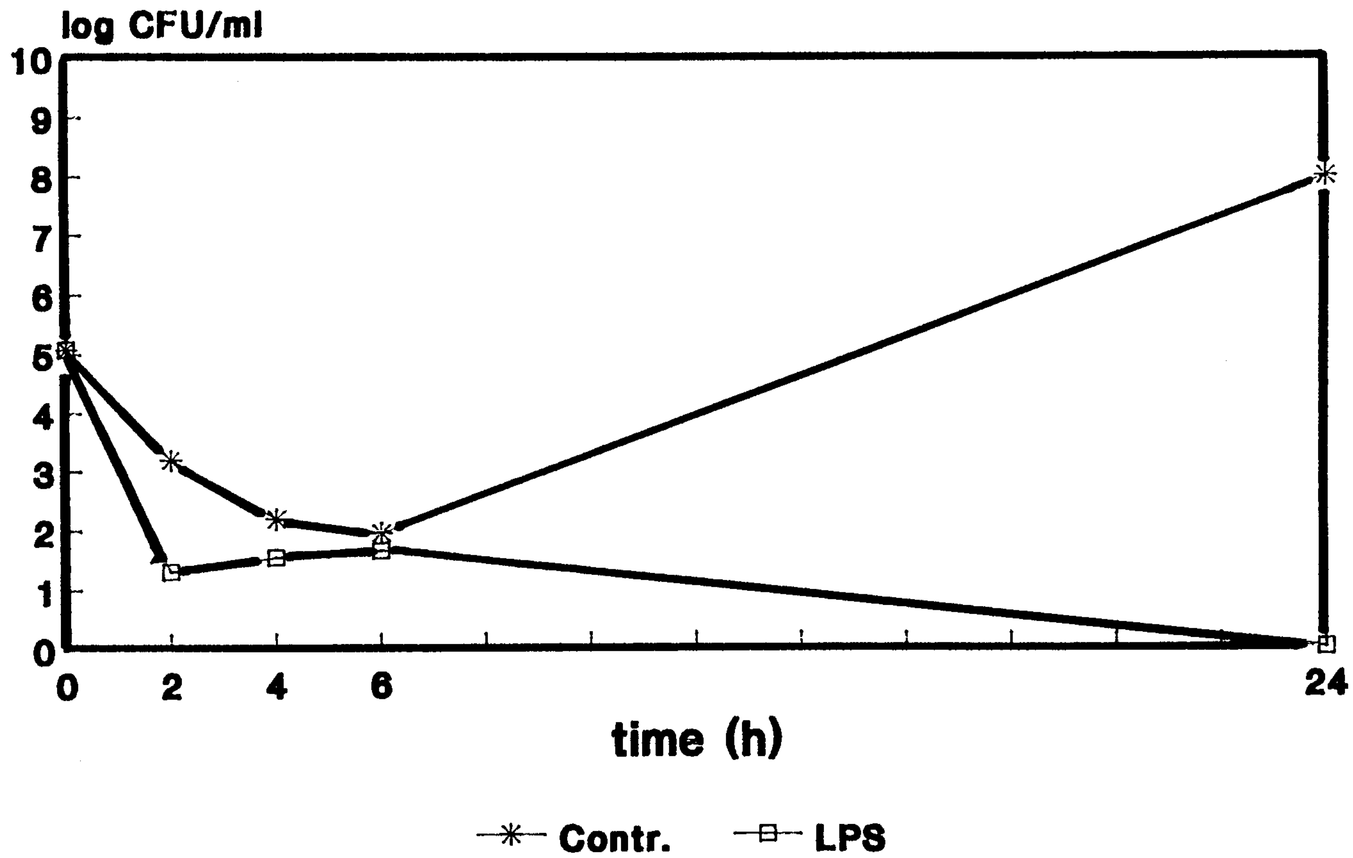


Figure 12

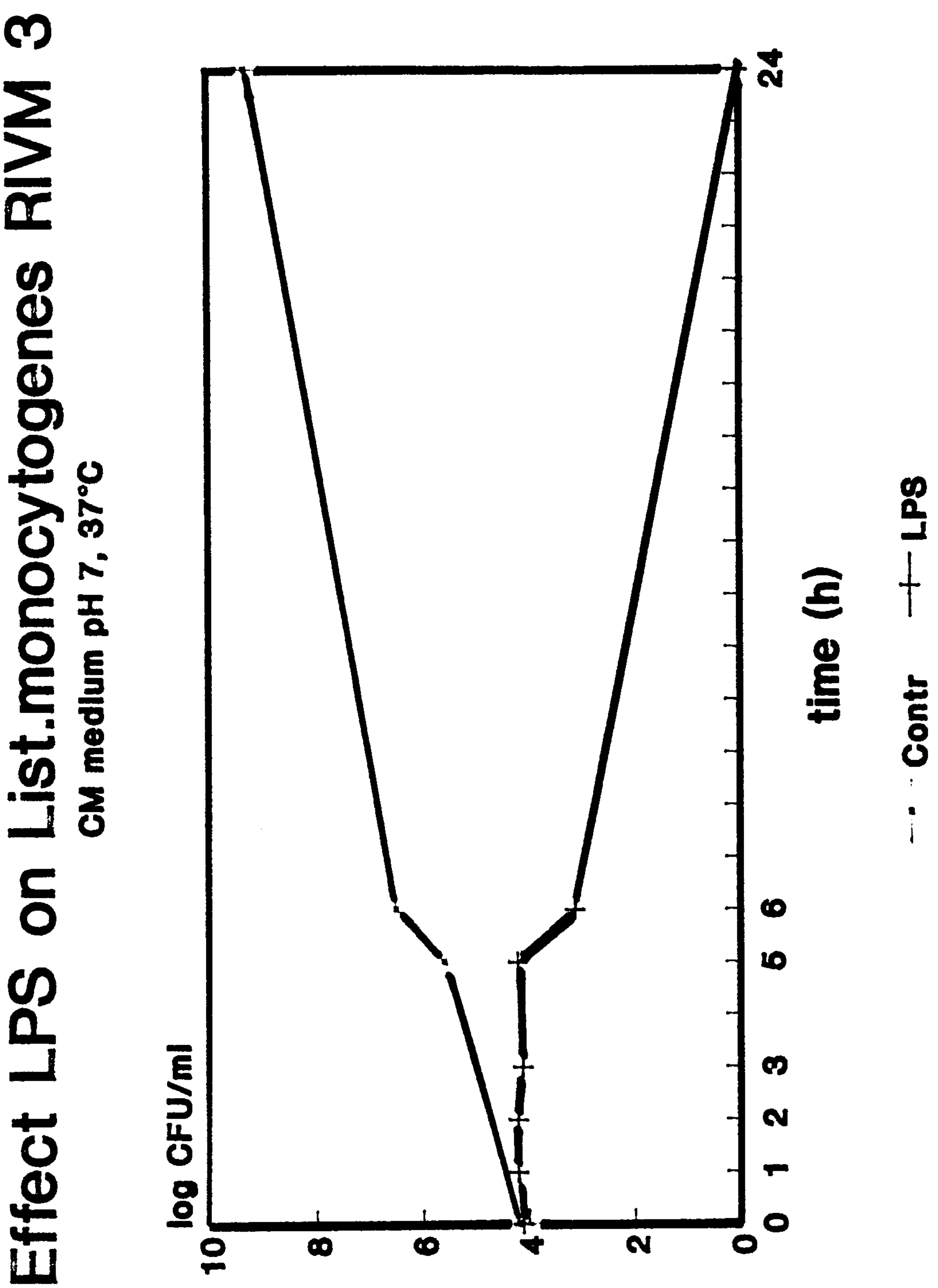
Effect LPS on List.monocytogenes RIVM 3
CM medium pH 7, 37°C
log CFU/ml
10
8
6
4
2
0
0
1
2
3
5
6
24
time (h)
Contr
LPS

Figure 13

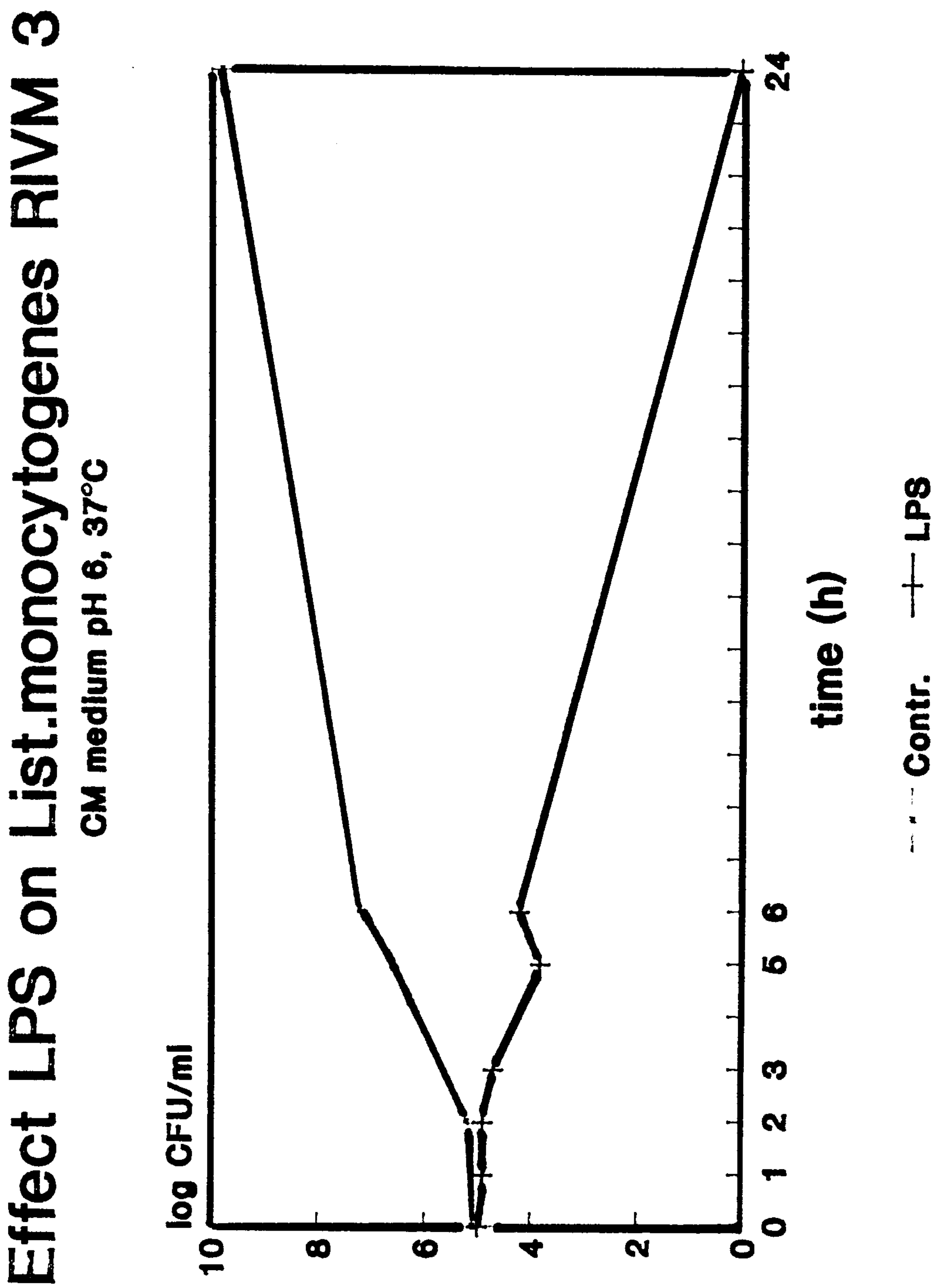
Effect LPS on List.monocytogenes RIVM 3
CM medium pH 6, 37°C
log CFU/ml
10
8
6
4
2
0
0
1
2
3
5
6
24
time (h)
Contr.
LPS

Figure 14

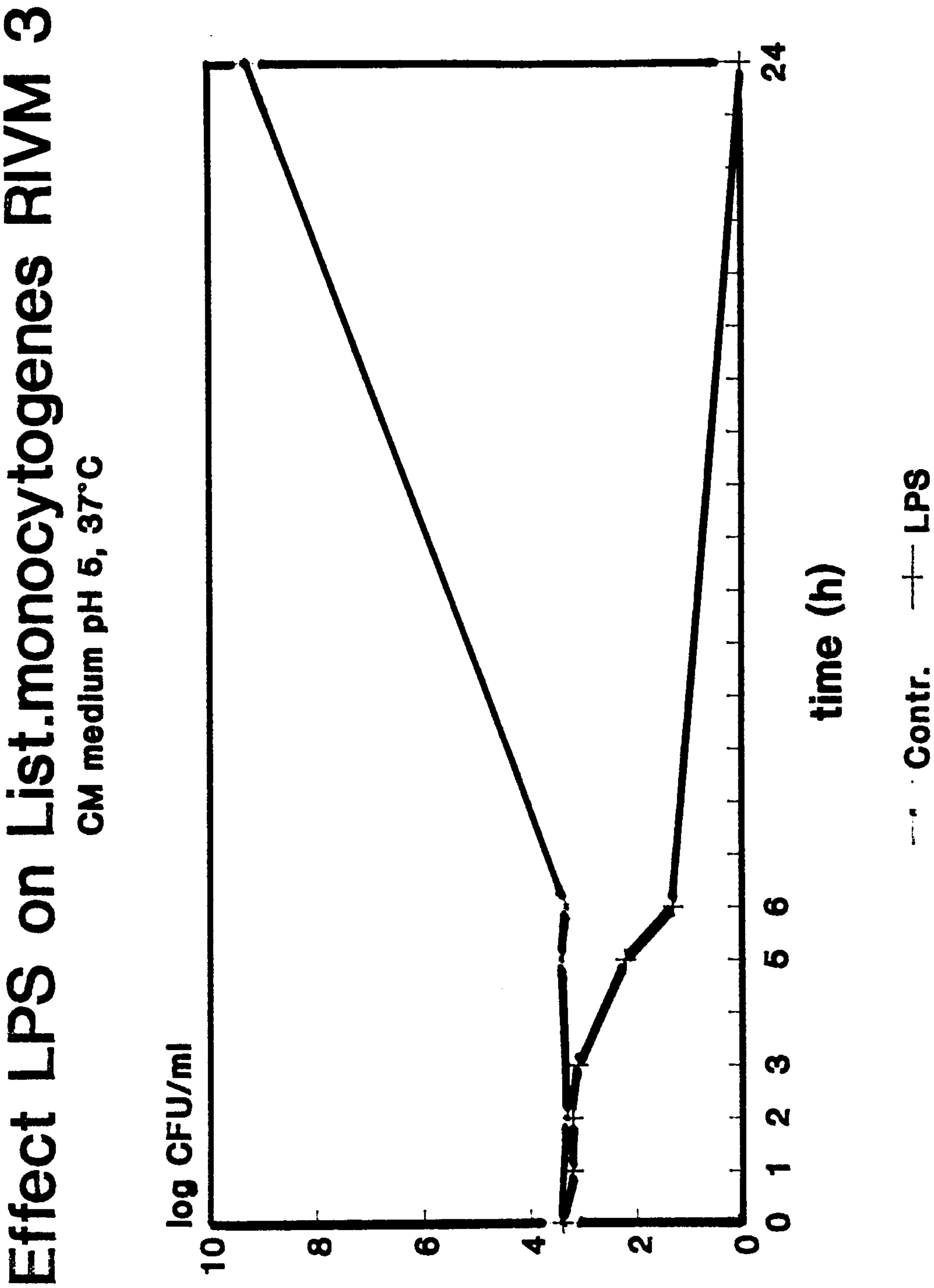
Effect LPS on List.monocytogenes RIVM 3
CM medium pH 5, 37°C
log CFU/ml
10
8
6
4
2
0
0
1
2
3
5
6
24
time (h)
Contr.
LPS

Figure 15

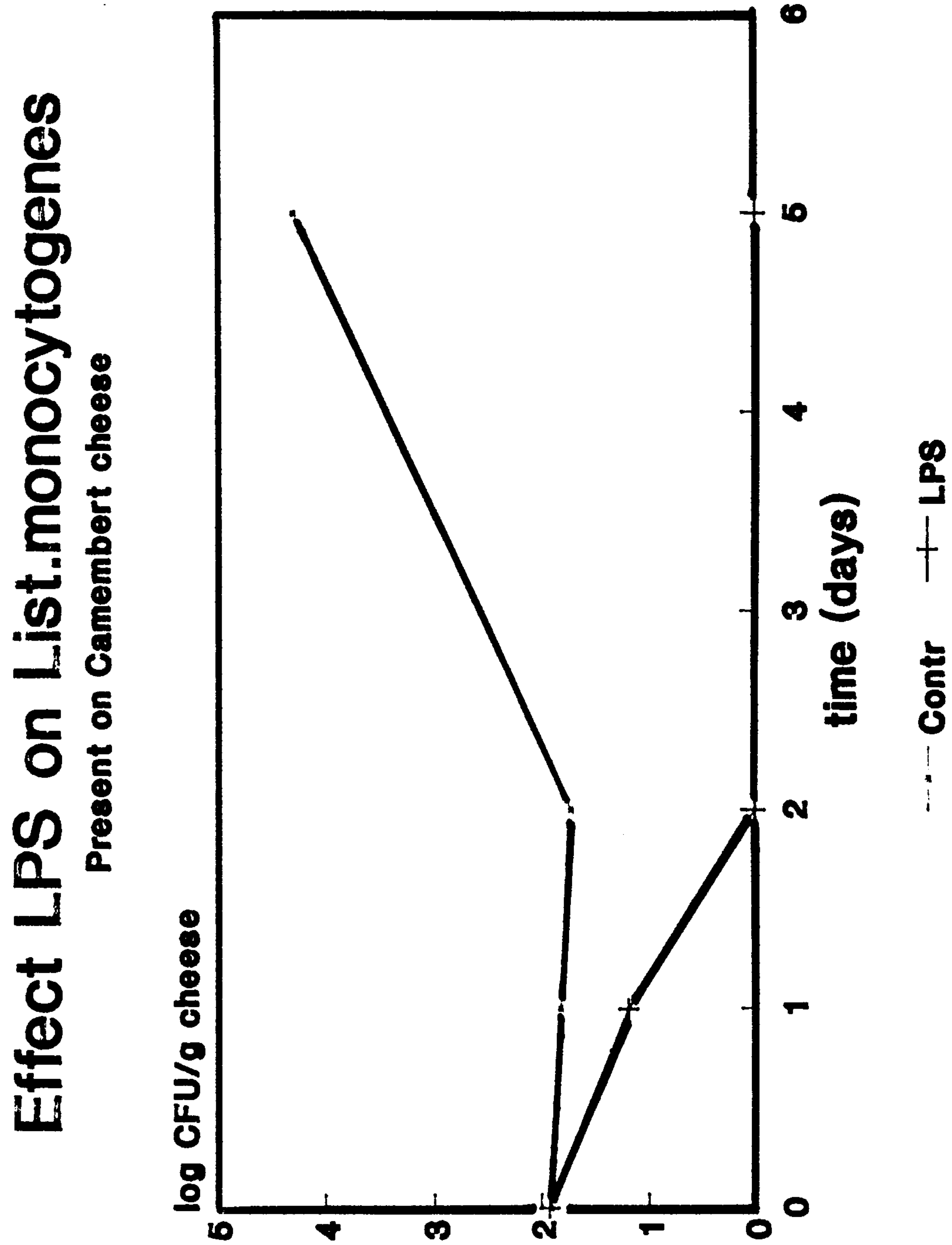
Effect LPS on List.monocytogenes
Present on Camembert cheese
log CFU/g cheese
5
4
3
2
1
0
0
1
2
3
4
5
6
time (days)
Contr
LPS

Figure 16

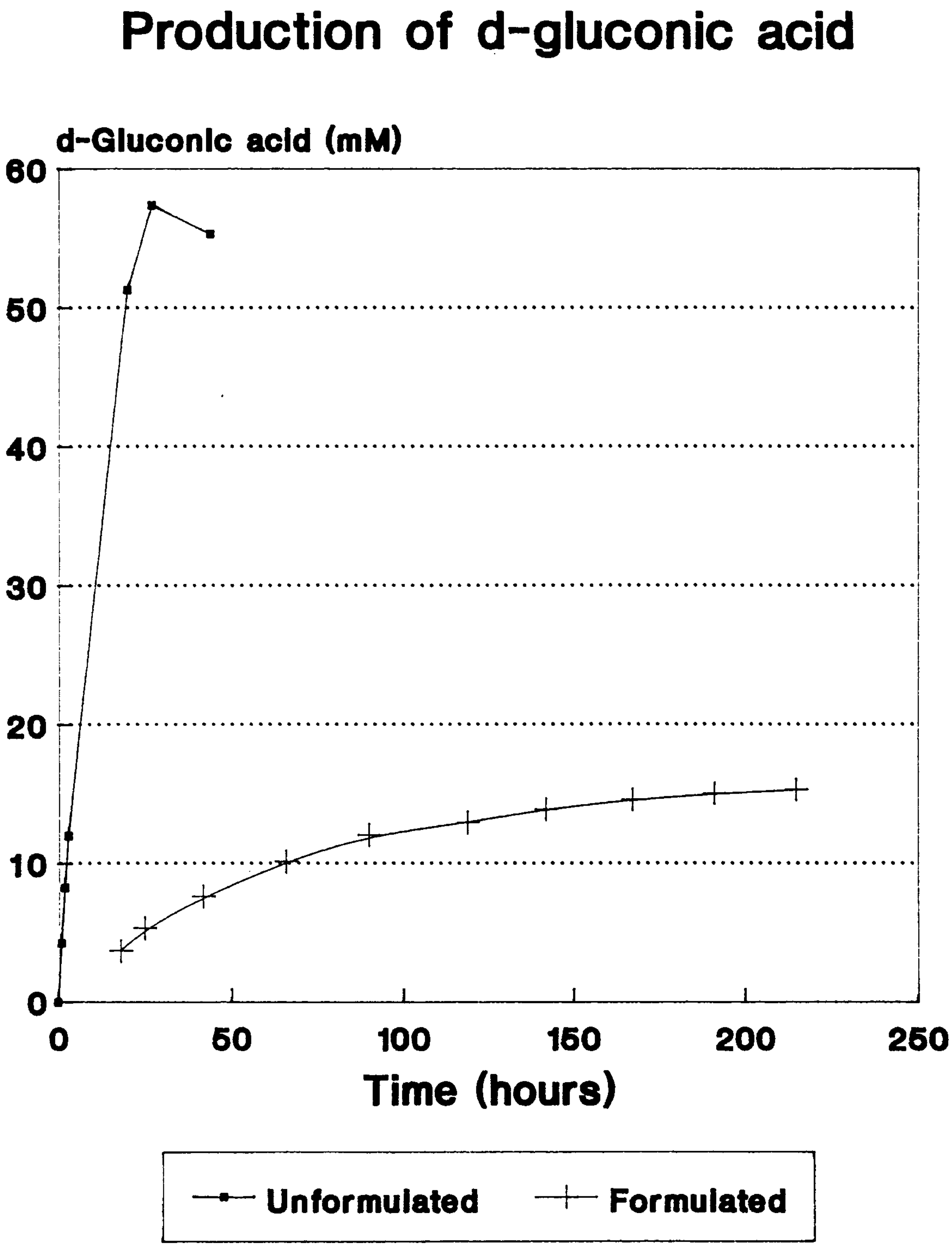
Production of d-gluconic acid
d-Gluconic acid (mM)
60
50
40
30
20
10
0
0
50
100
150
200
250
Time (hours)
Unformulated
Formulated

Figure 17

# PRODUCTION OF GLUCONIC ACID

**Product: 1 mg form./ml;**
**Buffer + 4.5 % NaCl pH 7.5; 7°C**

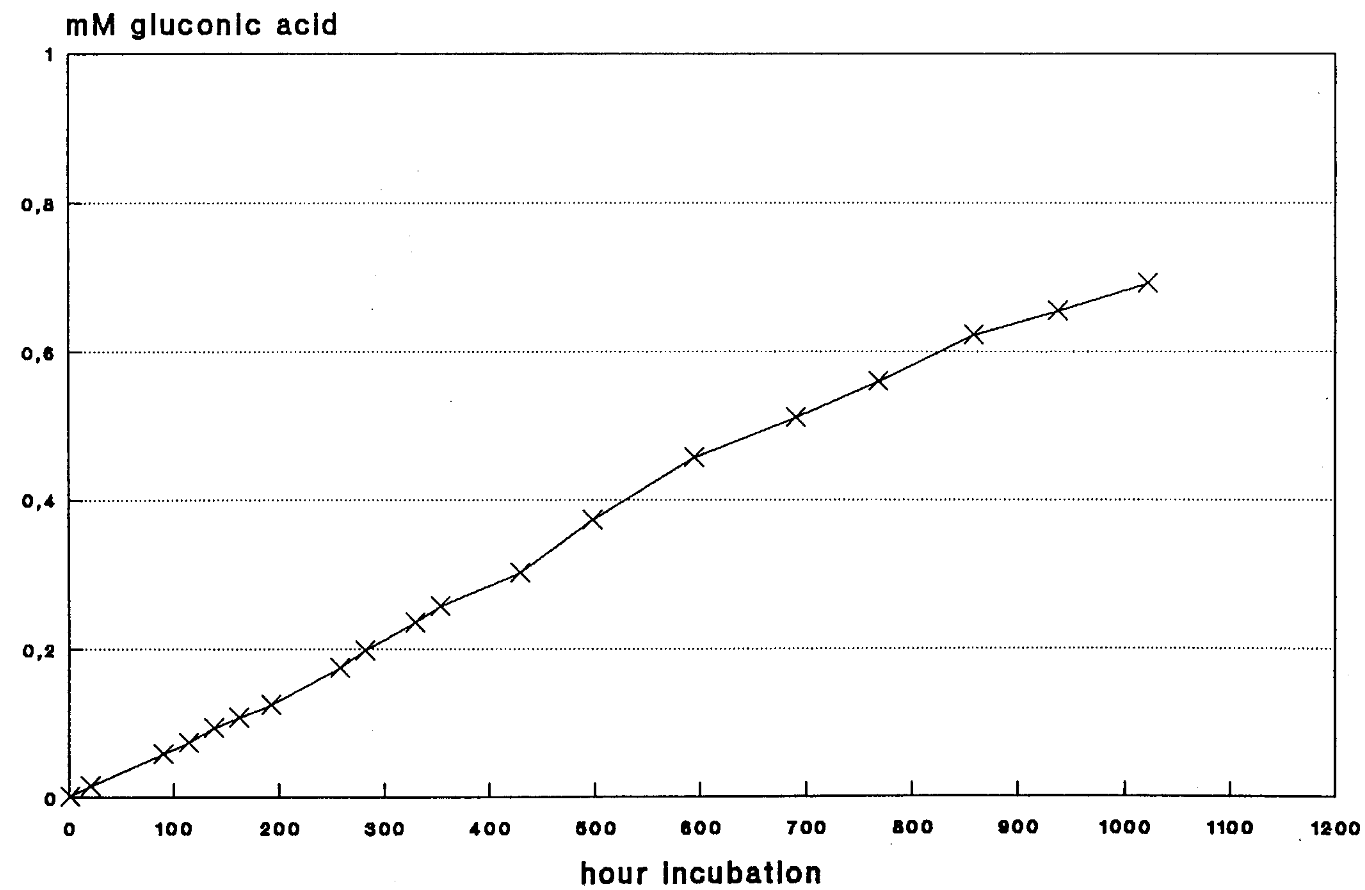


Figure 18